(19) Europäisches Patentamt
European Patent Office
Office européen des brevets

(11) **EP 4 541 262 A1**

(12) **EUROPEAN PATENT APPLICATION**

(43) Date of publication:
**23.04.2025 Bulletin 2025/17**

(21) Application number: **23203849.7**

(22) Date of filing: **16.10.2023**

(51) International Patent Classification (IPC):
**A61B 5/00** $^{(2006.01)}$    **G16H 30/40** $^{(2018.01)}$

(52) Cooperative Patent Classification (CPC):
**A61B 5/6852; A61B 5/7264; G16H 30/40;
G16H 50/20; G16H 50/50;** A61B 2576/023;
G16H 20/40

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **BIOSENSE WEBSTER (ISRAEL) LTD.
2066717 Yokneam (IL)**

(72) Inventors:
• **BARAM, Alon
Yokneam 2066717 (IL)**
• **BAR-TAL, Meir
Yokneam 2066717 (IL)**

(74) Representative: **Carpmaels & Ransford LLP
One Southampton Row
London WC1B 5HA (GB)**

(54) **LEFT ATRIUM SHAPE RECONSTRUCTION FROM SPARSE LOCATION MEASUREMENTS USING NEURAL NETWORKS**

(57)    A method includes, in a processor, receiving example representations of geometrical shapes of a given type of organ. In a training phase, a neural network model is trained using the example representations. In a modeling phase, the trained neural network model is applied to a set of location measurements acquired in an organ of the given type, to produce a three-dimensional model of the organ.

FIG. 3

**Description**

**FIELD OF THE INVENTION**

The present invention relates generally to cardiac mapping, and particularly to computation methods of anatomical cardiac maps.

**BACKGROUND OF THE INVENTION**

Catheter based radiofrequency ablation for pulmonary vein isolation has become the first line of treatment for atrial fibrillation in recent years. This requires a rather accurate map of the left atrial sub-endocardial surface including the ostia of the pulmonary veins, which requires dense sampling of the surface and takes more than 10 minutes. It is an object of the present invention to help provide left atrial visualization early in the procedure to ease procedure complexity and enable further workflows, such as using catheters that have difficulty sampling the surface.

Atrial fibrillation (AF), the most common form of cardiac arrhythmia in humans, affects millions of people worldwide annually. It is associated with increased risk of embolic stroke and decreased quality of life. Catheter based electro-anatomic-mapping (EAM) 3D guided radiofrequency ablation for pulmonary vein isolation (PVI) is rapidly becoming the first line of treatment.

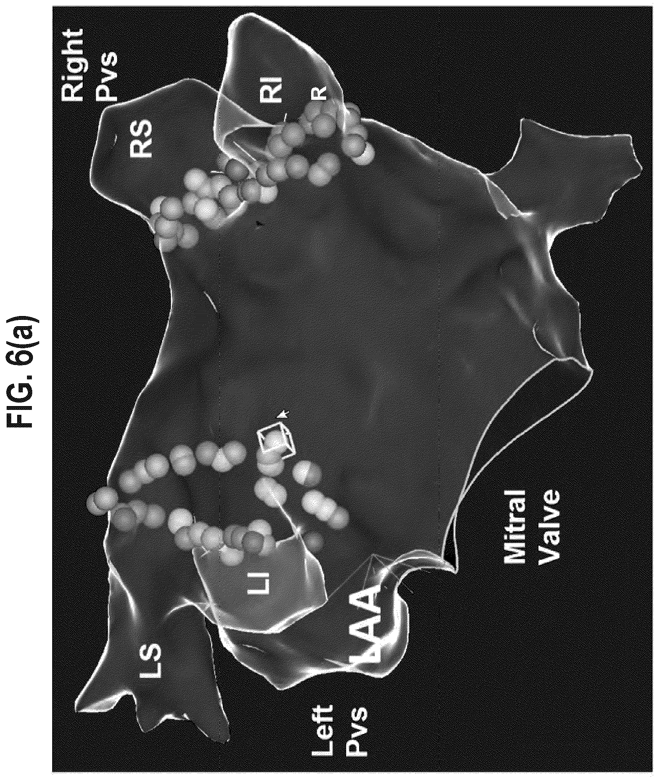

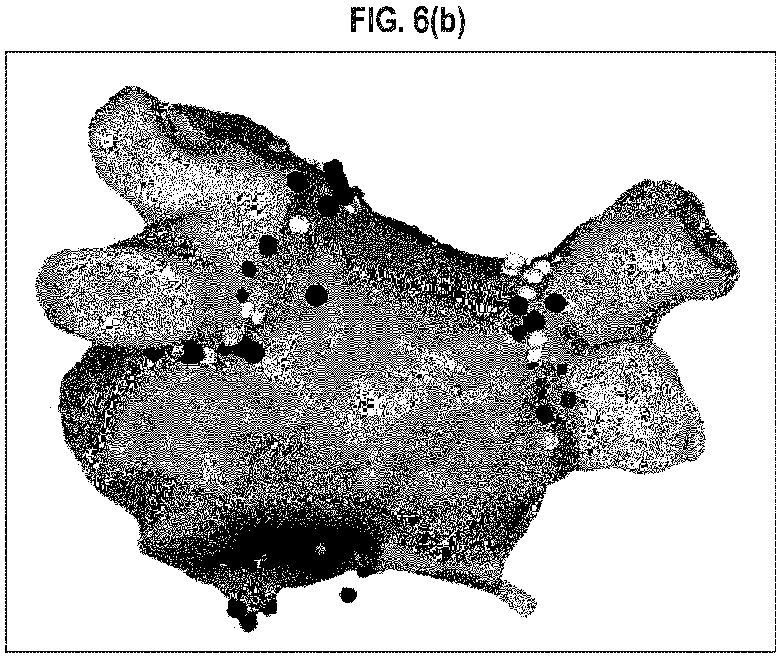

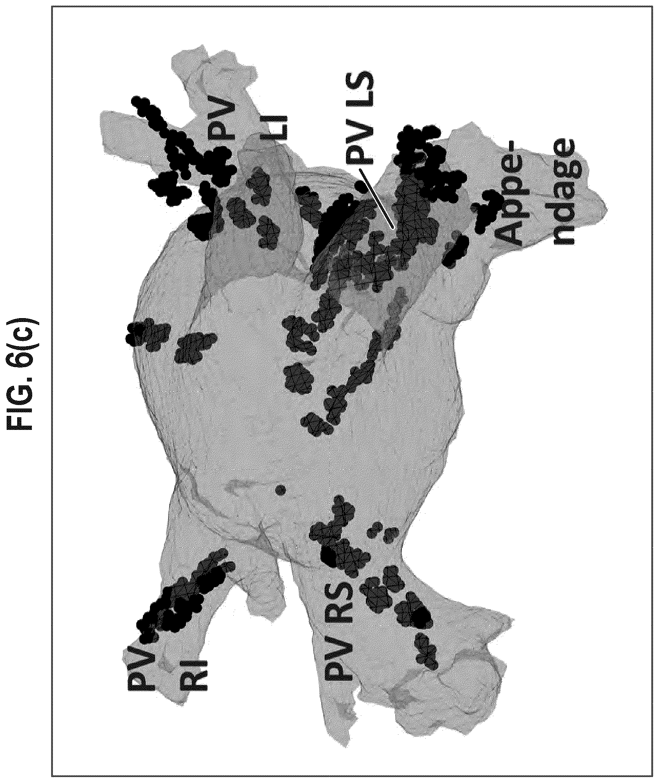

There are many other types of arrhythmia that are treated using catheter ablation some of which may appear during or after a PVI treatment for a significant portion of the cases. EAM systems record position and electrical signals acquired as the catheter is moved inside the chamber. Once a large enough number of points are accumulated, a geometric reconstruction algorithm reconstructs the endocardial surface. Using local activation times, the electrical propagation wave (over the surface) that causes chamber contraction is approximated. The procedure is schematically depicted in Figures 6A to 6C. The system provides visualization of the left atrial (LA) endocardial surface anatomy including the pulmonary veins (PV) anatomical parts (LS - left superior, RS - right superior, LI - left inferior, RI - right inferior, LAA - left atrial appendage).

Current EAM systems accumulate the locations traversed during the catheter's trip inside the LA and produce the surrounding shape as the boundary surface. The boundary extraction process is referred to as fast anatomical mapping (FAM). Producing an accurate anatomically correct LA surface requires the catheter to touch a large portion of the boundary (extracting many surface points). This requires extensive maneuvering of the catheter which requires physician skill, since little visual guidance is available, and takes tens of minutes of procedure time. The ablation itself is supported by local electrograms, force indications, and various indices showing whether the catheter is ablating in the right location. Some anatomical regions require special care or should be avoided completely when ablating such as the esophagus or deep inside the PVs.

Various imaging modalities such as magnetic resonance imaging (MRI), intracardiac ultrasonic catheters, etc. can be used for capturing the anatomical shape by segmenting the surface of the boundary from the acquired image data. Imaging systems must address issues such as acquisition time, radiation exposure, limited field of view (e.g. partially visited areas), noise and contrast, as well as the deformation of the heart shape due to breathing, heartbeat, and pose, in order to estimate the shape. A typical approach to segmentation is to transform the acquired image into a 3D map indicating the probability that a point belongs to the tissue or the blood pool. The resultant segmentation must comply with the constraint that the extracted shape be smooth. Prior knowledge of various anatomical details must also be integrated.

Anatomical imaging guidance can vary in scope and performance. Though findings are inconclusive as to the benefits of imaging methods (CT/MRI) in terms of the clinical outcomes of EP procedures, they do help reduce cognitive load, simplify the procedure and enable less proficient surgeons to achieve better results. The accurate imaging of difficult to reach areas of the atrium such as the ridge between the left superior vein and the left atrial appendage improve catheter navigation and the visualization of tissue contact during the actual ablation procedure.

Physicians can use different types of workflows when treating patients, as a function of the arrhythmia, the systems, and catheters available, level of proficiency, site regulations, etc. There are various types of catheters, each of which has a different shape and different parameters depending on whether it is used for diagnostic purposes or ablation, the type of arrhythmia, the patient, anatomy, etc. This affects catheter maneuverability, its ability to touch while not bending the surface, and the parts of the catheter whose location is tracked. It also directly impacts the accuracy of anatomical maps. Recently, single-shot catheters (large spherical "balloon" like) that can ablate a PV within seconds have become popular. However, they require guidance via fluoroscopy, CT/MRI, or the insertion of additional mapping catheters. A common workflow for a physician's initial bearing is traversing a path, namely "initial bearing path", that traverses known anatomical landmarks such as the four pulmonary veins ostia, and is acquired in under three minutes. An example of this path, alongside the corresponding anatomical shape can be seen in Figures 6A to 6C.

Some clinical procedures employ elaborate computation methods to generate an anatomical representation of an organ such as a cardiac chamber. For example, U.S.

Patent Application Publication 2014/0152653 describes methods for processing two-dimensional ultrasound images from an intracardiac ultrasound imaging catheter, which provide improved image quality and enable generating three-dimensional composite images of the heart. Two-dimensional ultrasound images are obtained and stored in conjunction with correlating information, such as time or an electrocardiogram. Images related to particular conditions or configurations of the heart can be processed in combination to reduce image noise and increase resolution. Images may be processed to recognize structure edges, and the location of structure edges used to generate "cartoon" rendered images of the structure. Structure locations may be averaged over several images to remove noise, distortions and blurring from movement.

As another example, U.S. Patent Application Publication 2017/0046616 describes a use of 3D deep convolutional neural network architecture (DCNNA) equipped with so-called subnetwork modules which perform dimensionality reduction operations on 3D radiological volume before the 3D radiological volume is subjected to computationally expensive operations. Also, the subnetworks convolve 3D data at multiple scales by subjecting the 3D data to parallel processing by different 3D convolutional layer paths. Such multi-scale operations are computationally cheaper than the traditional CNNs that perform serial convolutions. In addition, performance of the subnetworks is further improved through 3D batch normalization (BN) that normalizes the 3D input fed to the subnetworks, which in turn increases learning rates of the 3D DCNNA. After several layers of 3D convolution and 3D sub-sampling with 3D across a series of subnetwork modules, a feature map with reduced vertical dimensionality is generated from the 3D radiological volume and fed into one or more fully connected layers.

## SUMMARY OF THE INVENTION

The present invention utilizes an encoder-decoder network with a regularization term to reconstruct the shape of the left atrium from partial data which is derived from simple catheter maneuvers. To train the network, a dataset of 3D atria shapes can be acquired and corresponding catheter trajectories can be generated. Once trained, the network can sufficiently approximate the atrium shape based on a given trajectory. Such a network produces realistic visualization using partial acquisition within a 3-minute time interval.

There is provided, according to the present invention, a computer-implemented method, comprising: receiving a first dataset of points acquired from a catheter as it follows a path across a heart, each point in the first dataset comprising position data acquired at a certain position along the path in the heart; providing the first dataset to an encoder-decoder network , the encoder-decoder network trained based on training data comprising (1) a dataset of points representing a shape of a known heart and (2) a dataset of points representing a known catheter path across the known heart; outputting, with the encoder-decoder network, a second dataset of points, the second dataset representing a predicted, shape of the heart.

Such a process is advantageous, because it allows for a much smaller subset of data to be acquired for reconstructing a shape of a heart (which is useful for heart visualization for use in guiding future surgeries) as opposed to a more rigorous acquisition of an electroanatomical map (which requires placement of a catheter in an invasive procedure at significantly more positions across the heart to have an adequate resolution). The improved speed of this process means that a heart mapping procedure can be drastically shortened, which has benefits to patient comfort and reduces risk associated with lengthy surgical procedures.

A shape of a heart can refer to an entire heart, or to a portion of the heart, such as the left atrium.

An encoder-decoder network may refer to a specific architecture machine learning model (and specifically of a neural network). It comprises two stages (i.e. an encoder stage and a decoder stage). The encoder stage takes an input and processes the input into an encoded vector (i.e. a numerical representation of the data in the input), and then the decoder takes the encoded vector as an input and generates a output.

For the avoidance of doubt, whilst an encoder-decoder network can be utilized to form an autoencoder (whereby the encoder function and decoder function are reciprocal functions, so as to recreate identically an input at the decoder by the output of the decoder, and the model is trained via unsupervised learning), the present invention is not directed towards such embodiments. Instead, the present invention is directed towards non-autoencoder implementations of an encoder-decoder network, whereby the input data (in this case a sparse set of data acquired from a path along a heart) and the output data (in this case a reconstructed shape of the heart) differ from one another (and thus, the encoder function and the decoder function are not directly reciprocal). For simplicity, where the disclosure discusses an encoder-decoder network, this refers to non-autoencoder implementations.

It should be understood that training a machine learning model in a supervised way refers to providing a labelled data set to the machine learning model, so that the model can initialize one or more parameters of the model and thereby "learn" a relationship between its input (i.e. elements in the data set) and its output (i.e. the label of the respective element).

In other words, the encoder-decoder network can be trained through a supervised learning method, whereby data pairs are provided to the network (a first set of data provided to the input, and corresponding labels for the first set of data provided to the output). It may be understood that in this case, the dataset of points representing a known catheter path across the known heart in the training data acts as the input data set, whereas the dataset of points representing a shape of a known heart acts as labels for the input data set.

Thus, the encoder-decoder network can learn to take a dataset of points representing a known catheter path, and predict a shape of the heart.

The process of training may include jointly training the encoder and the decoder in the encoder-decoder network, through any known training technique (e.g. back propagation and teacher forcing).

The dataset of points acquired in the present invention can be acquired from a digital memory (e.g. based on data collected at an earlier time), so that the reconstruction process can occur entirely distinctly from the catheterization process.

According to one embodiment of the invention, receiving the first dataset of points step comprises receiving the first dataset of points from a memory.

As noted above, the dataset of points acquired in the present invention can be acquired from a digital memory (e.g. based on data collected at an earlier time), so that the reconstruction process can occur entirely distinctly from the catheterization process.

According to one embodiment of the invention, the encoder-decoder network is a dense encoder-decoder network.

It may be understood that a "dense" neural network refers to a network in which the neurons in one or more (e.g. all) layers of the network are connected to each and every neuron of the preceding layer.

According to one embodiment of the invention, the method further comprises reconstructing a shape of the heart based on the predicted second dataset of points in the heart.

By reconstructing a shape based on the second dataset of points, a more easily visualized heart, based on fewer datapoints, can be presented to the surgeon. In some instances, this reconstruction can include presenting the reconstructed shape on a display.

According to one embodiment of the invention, the path across the heart is a path across a left atrium, wherein the known catheter path across the known heart is a path across a known left atrium, and wherein the shape of the known heart is a shape of at least a portion of the known left atrium.

According to one embodiment of the invention, the path and the known catheter path each follow a certain trajectory through the left atrium.

According to one embodiment of the invention, each trajectory includes a path starting from the septum, and continuing to left inferior, right inferior, and right superior in order.

For the avoidance of doubt, the encoder-decoder network trained on a certain set of data should be applied to corresponding data to ensure its accuracy. Thus, the catheter path used for training should match the catheter path taken during acquisition. Whilst the specific direction of the path is not essential, it is advantageous to have a catheter path in which the catheter is brought into proximity with a number of landmarks in the left atrium (e.g. the septum).

According to one embodiment of the invention, the training of the encoder-decoder network comprises minimizing a loss function, and wherein the loss function comprises a cross entropy loss term.

It may be understood that a cross-entropy loss term refers to a portion of a loss function having contrast between two random variables. It may be, e.g. a binary cross-entropy loss function, which is used in binary classification tasks. It may also be referred to as a logarithmic loss.

According to one embodiment of the invention the loss function comprises a linear combination of the cross entropy loss term and a negative of a Sorenson-DICE coefficient.

A Sorenson-Dice coefficient, otherwise known as Dice's coefficient, refers to a statistic for gauging the similarity of two samples. It may be defined as being equal to $\frac{2|X \cap Y|}{|X|+|Y|}$, where X and Y are the two samples respectively.

According to one embodiment of the invention, the Sorenson-Dice coefficient is weighted with a weighting mask parameter, the weighting mask parameter configured to control the sensitivity of the model around the surface boundary of the heart.

The weighting mask parameter may be a weight given from 0 to 1, which can be used as a multiplicative factor for each voxel, and therefore allows the model to treat the boundary of the heart (which is critical for shape reconstruction) differently from the interior of the heart.

According to one embodiment of the invention, the loss function further comprises a regularization term, the regularization term configured to control the smoothness of the shape of the heart.

According to one embodiment of the invention, the regularization function comprises derivatives of weights of a first layer of the encoder-decoder network, and preferably derivates of weights of the first layer and the output layer of the encoder-decoder network.

According to one embodiment of the invention, the training of the encoder-decoder network comprises utilizing an Adam optimization algorithm.

An Adam optimization algorithm may refer to an adaptive moment estimation, which is a version of a stochastic gradient dissent method. This algorithm takes advantage of second moments of the gradients. It calculates an exponential moving average of gradients and square gradients, and includes parameters for controlling the decay rate of these moving averages. Accordingly, the Adam optimization algorithm is a combination of two gradient descent methods: an Adaptive Gradient Algorithm (AdaGrad) and a Root Mean Square Propagation (RMSProp).

Such an algorithm is beneficial in deep learning because of the speed at which the training can be performed.

According to one embodiment of the invention, the encoder-decoder network comprises a first encoder and a first decoder, and wherein the encoder-decoder network comprises a plurality of layers, selected from one or more of an input layer, one or more hidden layers, and an output layer.

According to one embodiment of the invention, the encoder decoder network comprises one or more of the following:

tied weights between the first encoder and the first decoder;
a masked input;
an RELU activation function in each layer in the encoder-decoder network except for the output layer;
a sigmoid activation function in the output layer;
a batch-normalization layer following each layer in the encoder-decoder network except for the last layer.

Tying weights can refer to a technique whereby weights of a decoder are set to the same as the weights of the encoder. In this instance, the first encoder and the first decoder can have weights tied together, although further encoders and decoders can be present in the system and may not require tied weights.

A masked input allows for variable input lengths to be received, because the masked input forces fixed length inputs to be received by the encoder-decoder network.

An activation function in machine learning refers to a function that calculates the output of a node (based on the nodes inputs and weights of individual inputs). Different activation functions can be used to perform different effects (e.g. ridge functions, radial functions, and fold functions). An activation function can be applied to each node in a certain layer of the machine learning model.

A ReLU (or rectifier) activation function can refer to a function defined as the positive part of its argument $\sigma(x) = \max(0, x)$, where x is the input to the neuron. This is also known as a ramp function.

A sigmoid activation function can refer to $\sigma(x) = 1/(1+e^{-x})$, where x is the input to the neuron.

A batch-normalization layer may refer to a layer which applies a transform to normalize its inputs (e.g. maintain a mean close to 0 and a standard deviation close to 1).

According to one embodiment of the invention, at least one point in the dataset corresponds to a position in the blood-pool where the catheter is not touching the surface of the heart.

According to one embodiment of the invention, every point in the dataset corresponds to a position in the blood-pool where the catheter is not touching the surface of the heart.

When points in the dataset can be gathered from the blood pool, as opposed to pressed into tissue, a much faster data acquisition process can be used. That is, because electrical data does not need to be gathered during the process of the present invention, a surgeon can merely navigate the catheter through the heart and collect location data at a plurality of points along the path. The absence of a requirement to collect/transmit electrical data means that the catheterization process is much quicker than for more labor-intensive electrical mapping procedures.

According to one embodiment of the invention, the training data dataset of points representing the known catheter path across the known heart comprises added noise and the trained encoder-decoder network is configured to remove noise from the first dataset of points.

In some instances, additional random noise (e.g. white noise) can be added to the dataset, so that the encoder-decoder network can be trained how to remove said noise (thus improving the predictions of the encoder-decoder network).

In another aspect of the invention, there is provided a system comprising:

a memory, which is configured to store a first dataset of points acquired from a catheter as it follows a path across a heart, each point in the first dataset comprising position data acquired at a certain position along the path in the heart; and
a processor which is configured to:

receive, from the memory, the first dataset of points;
provide the first dataset to an encoder-decoder network, the encoder-decoder network trained based on training data comprising (1) a dataset of points representing an shape of a known heart and (2) a dataset of points representing a known catheter path across the known heart;

output, with the encoder-decoder network, a second dataset of points, the second dataset representing a predicted, shape of the heart.

According to one embodiment of the invention, the encoder-decoder network is a dense encoder-decoder network.

According to one embodiment of the invention, the processor is further configured to reconstruct a shape of the heart based on the predicted second dataset of points in the heart.

According to one embodiment of the invention, the path across the heart is a path across a left atrium, wherein the known catheter path across the known heart is a path across a known left atrium, and wherein the shape of the known heart is a shape of at least a portion of the known left atrium.

According to one embodiment of the invention, the path and the known catheter path each follow a certain trajectory.

According to one embodiment of the invention, each trajectory includes a path starting from the septum, and continuing to left inferior, right inferior, and right superior in order.

According to one embodiment of the invention, the training of the encoder-decoder network comprises minimizing a loss function, and wherein the loss function comprises a cross entropy loss term.

According to one embodiment of the invention, the loss function comprises a linear combination of the cross entropy loss term and a negative of a Sorenson-DICE coefficient.

According to one embodiment of the invention, the Sorenson-Dice coefficient is weighted with a weighting mask parameter, the weighting mask parameter configured to control the sensitivity of the model around the surface boundary of the heart.

According to one embodiment of the invention, the loss function further comprises a regularization term, the regularization term configured to control the smoothness of the shape of the heart.

According to one embodiment of the invention, the regularization function comprises derivatives of weights of a first layer of the encoder-decoder network, and preferably derivatives of weights of the first layer and the output layer of the encoder-decoder network.

According to one embodiment of the invention, the training of the encoder-decoder network comprises utilizing an Adam optimization algorithm.

According to one embodiment of the invention, the encoder-decoder network comprises a first encoder and a first decoder, and wherein the encoder-decoder network comprises a plurality of layers, selected from at least one of an input layer, one or more hidden layers, and an output layer.

According to one embodiment of the invention, the encoder decoder network comprises one or more of the following:

tied weights between the first encoder and the first decoder;
a masked input;
an RELU activation function in each layer in the encoder-decoder network except for the output layer a last layer in the plurality of layers;
a sigmoid activation function in the last layer;
a batch-normalization layer following each layer in the encoder-decoder network except for the last layer.

According to one embodiment of the invention, at least one point in the dataset corresponds to a position in the blood-pool where the catheter is not touching the surface of the heart, optionally where every point in the dataset corresponds to a position in the blood-pool where the catheter is not touching the surface of the heart.

According to one embodiment of the invention, the training data dataset of points representing the known catheter path across the known heart comprises added noise such that the trained encoder-decoder network is configured to remove noise from the first dataset of points.

There is also provided a computer-implemented method of generating training data to train a neural network to predict a dataset of points representing a shape of a heart given a dataset of points representing a catheter path across the heart, the method comprising: generating one or more synthetic atria shapes by (1) acquiring a predefined heart shape model representing a shape of an atrium; (2) generating, from the predefined heart shape model, a statistical prior model, the statistical prior model modeled as a multivariate normal distribution; (3) generating one or more atria shape samples from the statistical model, each atria shape sample having a given statistical score from the statistical model; (4) retaining a first set of atria shape samples from the statistical model based on the respective statistical score meeting a threshold; and (5) generating one or more synthetic atria paths for each atria shape sample in the first set, by locating an ostium of each pulmonary vein in the atrium and determining a path between each of the ostia given a predefined traversal order via solving a graph optimization problem.

Through such a technique, a consistent dataset used for training a neural network can be generated without the need for a repository of anatomical shapes and catheter paths within hearts (which would also be entirely suitable for reproducing the neural network). Any number of heart shapes can be generated from the predefined heart shape model, and for each heart shape generated, any number of synthetic atria paths can be further generated and associated with the respective heart shape. This provides a set of labelled data which can be used for training (i.e. the catheter path as an input, and the heart shape as the label/output used in training).

The present disclosure also describes a method, including, in a processor, receiving example representations of geometrical shapes of a given type of organ. In a training phase, a neural network model is trained using the example representations. In a modeling phase, the trained neural network model is applied to a set of location measurements acquired in an organ of the given type, to produce a three-dimensional model of the organ.

The present disclosure also describes a system, including a memory and a processor. The memory is configured to store example representations of geometrical shapes of a given type of organ. The processor is configured to (a) upload the example representations from the memory, (b) in a training phase, train a neural network model using the example representations, and store the trained neural network model in the memory, and (c) in a modeling phase, apply the trained neural network model to a set of location measurements acquired in an organ of the given type.

The present invention will be more fully understood from the following detailed description of the embodiments thereof, taken together with the drawings in which:

## BRIEF DESCRIPTION OF THE DRAWINGS

Fig. 1 is a schematic, pictorial illustration of a system for electro-anatomical mapping, in accordance with an embodiment of the present invention;

Figs. 2A and 2B are schematic, pictorial illustrations of regions over a left atrium where sets of locations were measured, in accordance with an embodiment of the present invention;

Fig. 3 is a block diagram that schematically illustrates a system for reconstructing a shape of a left atrium using a trained neural network model, in accordance with an embodiment of the present invention;

Fig. 4 is a flow-chart that schematically illustrates a method for reconstructing a shape of the left atrium using neural networks, in accordance with an embodiment of the present invention; and

Fig. 5 is a schematic, pictorial illustration of a left atrium shape reconstruction of a laboratory phantom, in accordance with an embodiment of the present invention.

Figs. 6A to 6C are schematic depictions of a left atrium surface acquired via a catheter based electro-anatomic-mapping (EAM) procedure.

Fig. 7 is a depiction of simulated generation of a heart shape with corresponding catheter paths.

Figs. 8A to 8C depict a synthetic catheter path creation process.

Fig. 9 depicts a relationship between voxels in space and neurons in a layer of the encoder-decoder network.

Figs. 10A and 10B depicts a visualization of the weights from the first layer of the encoder-decoder network.

Figs. 11A and 11B depicts a visualization of the boundary enhancement mask.

Fig. 12A depicts a focal catheter point cloud path.

Fig. 12B depicts a focal catheter voxelized path.

Fig. 12C depicts a lasso catheter voxelized path.

Fig. 12D depicts a point cloud of a focal catheter registered with a path of the mean shape.

Figs. 13A and 13B depicts reconstruction results comparing different amounts of an SWR against a Vnet solution.

Fig. 14 depicts a comparison of SWR005 and mean shape surface to surface distance for several radii (in mm).

Figs. 15A and 15B depict a comparison of reconstruction results focusing on the location and orientation of the PVs, for 2 sample cases.

Fig. 16 depicts a visualization of the reconstructions in the clinical CT cases for the SWR005, SWR75, V-Net, and the mean shape.

Figs. 17A to 17C depicts invalid anatomy in V-Net reconstructions.

Figs. 18A and 18B depicts a comparison of FAM and DED reconstructions using the path of the initial bearing.

Figs. 19A and 19B depict an illustration of a local PV search.

Figs. 20A and 20B depict the effects of path weighting.

## DETAILED DESCRIPTION OF EMBODIMENTS

OVERVIEW

A cardiac chamber, such as a left atrium, has a geometrically complex shape that may be electro-anatomically mapped in a partial manner during a mapping procedure, such as using catheter-based anatomical mapping. Methods for representing a left atrium from electro-anatomical data are provided, for example, in U.S. Patent 9,576,107, which uses a physical model together with a statistical model, and whose disclosure is incorporated herein by reference. However, representations provided by existing methods tend to be noisy, and as noted above, only partial. In the present context, the term "partial" means that entire regions of the left atrium are not mapped. In such cases, naive interpolation or extrapolation of existing location measurements are practically useless.

Embodiments of the present invention that are described hereinafter utilize a database made of example representations of geometrical shapes of a given type of an organ, such as shapes of left atria of hearts of multiple different subjects, to (i) train a neural network model using the example representations, and (ii) in a modeling phase of a particular heart, apply the trained neural network model to a set of location measurements acquired in a left atrium of the particular heart in order to produce a three-dimensional model of that left atrium.

In some embodiments, the database of example representations of left atria shapes (i.e., representations of surfaces that represent each a boundary between blood pool and heart tissue are reconstructed from) is based on location measurements performed by a measurement system such an electro-anatomical mapping system and/or a medical imaging modality.

In some embodiments, the database of example representations of left atria shapes is constructed by processing medical imaging studies comprising a series of images, such as from Computerized Tomography (CT), Magnetic Resonance Imaging (MRI) and/or Ultrasound (US) modalities, using appropriate software. In an embodiment, the representations are extracted from medical images of portions of left atria using a sphere intersection model, as described below.

In some embodiments, in the modeling phase, a set of locations over a region of an inner surface of an organ, such as a region of the left atrium, is electro-anatomically measured. The acquisition occurs as a physician maneuvers a catheter, for example, from the trans-septal entry point to touch the major left and right pulmonary veins (PV) for initial anatomy orientation of the atrium. The catheter path performed by the physician may contain more information, as the physician usually slides the catheter across path boundaries to reach the PVs.

Based on the acquired locations, the disclosed trained neural network technique produces a three-dimensional model of the left atrium of the particular heart being investigated, i.e., completing the measured location data into a more complete representation of the left atrium, as described below.

The present invention addresses the task of mapping the endo-cardial surface of the LA using a portion of the catheter traversal path, to provide the physician with early visualization, and reduce the mapping time while maintaining anatomical accuracy, especially in the PV ostia. This can also provide guidance for catheters that have difficulties sampling the surface such as balloon catheters.

Such a system can be trained for the LA anatomy variation with 4 PVs which corresponds to the majority of the population. The openings and orientations of the PVs reconstructed by such a system has minimal errors, and the anatomical parts are easily identifiable.

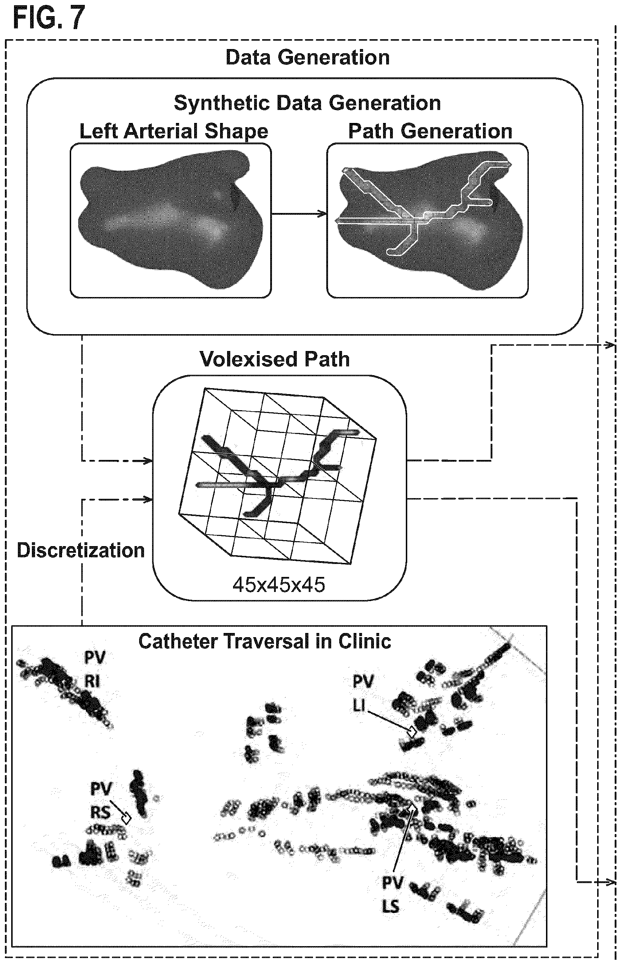

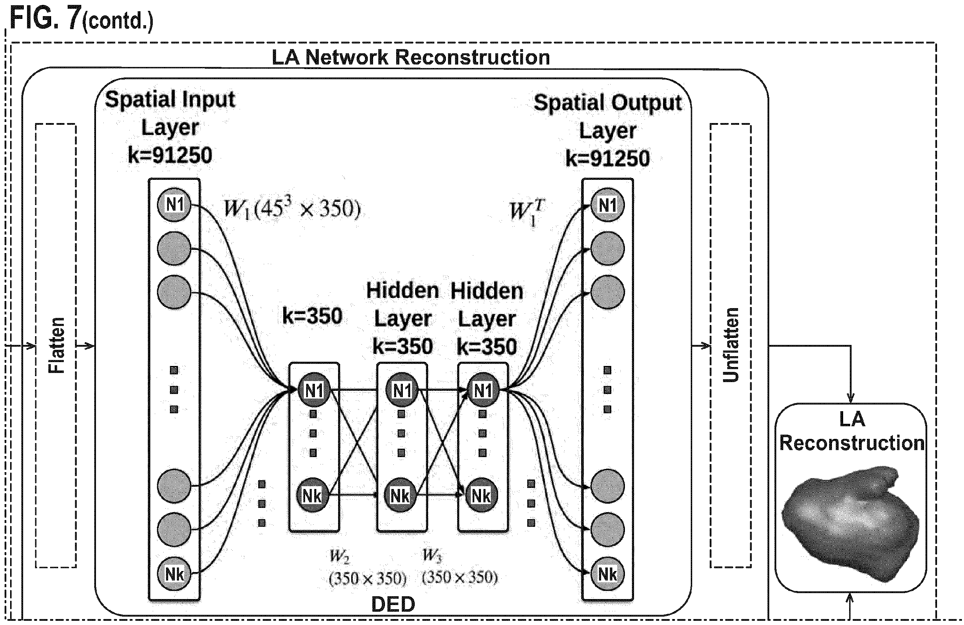

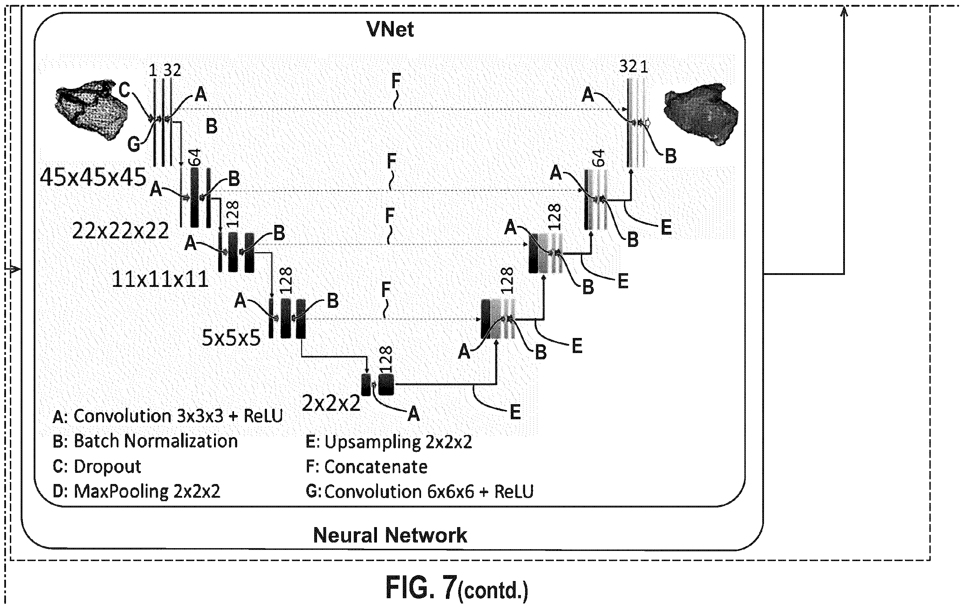

One solution is composed of two main steps: a path generation step, and an atria-shape reconstruction step. These are depicted in Figure 7. In the first step, paths resembling the path of the initial bearing to create a dataset for training are generated. Note that the sequence of the acquired path is not essential, only the spatial locations of the points. Those are the input to a neural network whose goal is to provide anatomy surface reconstruction of the LA according to the criteria mentioned above.

Supervised neural network-based algorithms require large tagged data sets. In this case, the training set should consist of meaningful relevant samples that allow for de-noising and the reconstruction of the LA shape from partial, noisy information. Unfortunately, a large enough set of patient atrial data, usually from CT or MRI, is not easily accessible. In order to address this issue, a Left-Atrium generator, developed by Biosense Webster, can be utilized that can generate instances of LA anatomical shapes. The present invention also provides an algorithm to create clinically feasible simulated sparse catheter paths, for each such generated LA shape. It is shown in experiments that the path generation process provides a more robust solution for real-world catheter path scenarios.

In a follow-up reconstruction task, the LA shape is reconstructed from a given catheter path input. A network-based solution is used, that receives a corrupted data-set of points as its input and is trained to predict the original, uncorrupted full data-set of points as its output. In an embodiment, the network used is a dense encoder-decoder network (hereon referred to as the DED solution), as shown in Figure 7. The DED loss incorporates a novel regularization term that enables the learning of smooth shapes. Since the catheter paths performed in the clinic differ from the synthetic ones, an enhanced surface boundary loss is introduced, improving solution robustness.

The DED network accuracy is 5mm. This outperforms a standard mean-shape solution in which the mean atria is rigidly transformed to match the input. The proposed solution also provides anatomically relevant outputs, whereas a standard V-Net architecture solution does not. Finally, the mapping time required for the reconstruction is less than 3 minutes. This enables a shape visualization at a much earlier time in the procedure as compared to existing solutions.

Additional contributions of this application include:

- Generation of a training dataset that is comprised of LA shapes and corresponding paths.
- Reconstruction methodology that takes a partial catheter path as input and is able to reconstruct a clinically viable LA shape as its output. Novel input augmentation and network loss functions are used to increase the robustness of the solution to the noisy (partial) synthetic catheter paths used in the training process.
- The overall solution proposed provides early visualization and guidance even for catheters that are difficult to map with.

The disclosed techniques reconstruct a realistic, and clinically valuable, shape of a cardiac chamber (e.g., a left atrium) from a sparse set of measured locations. By doing so, the disclosed technique and system may assist a physician in planning a proper treatment, such as a cardiac ablation. Since the disclosed techniques utilize only a sparse set of measurements, the mapping procedure may be shortened and simplified.

SYSTEM DESCRIPTION

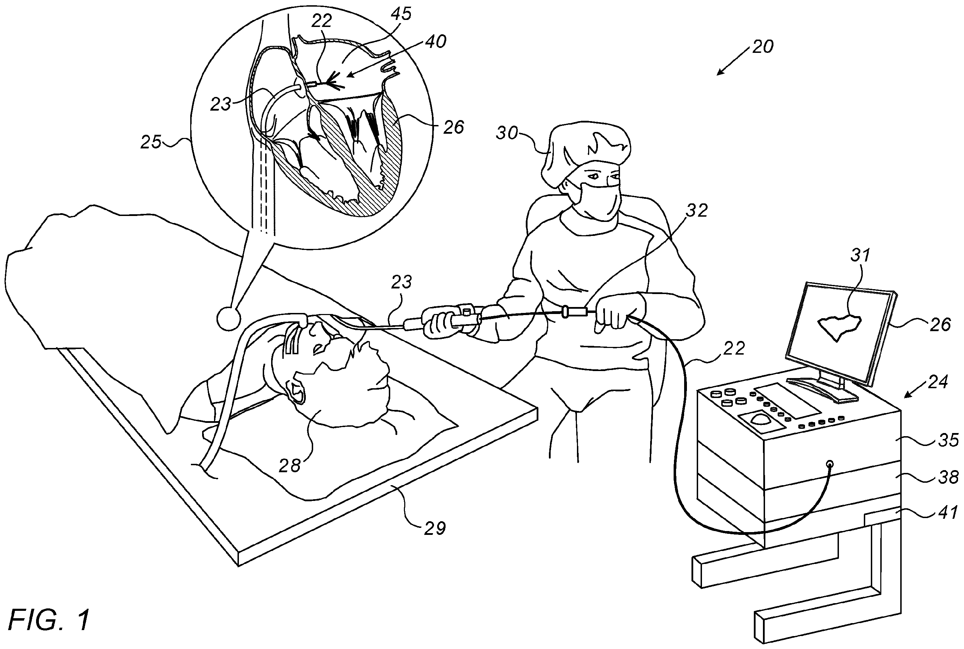

Fig. 1 is a schematic, pictorial illustration of a system 20 for electro-anatomical mapping, in accordance with an embodiment of the present invention. Fig. 1 depicts a physician 30 using an electro-anatomical catheter 40 to perform an electro-anatomical mapping of a cardiac chamber, such as a left atrium 45, of a heart 26 of a patient 28 laying on a table 29. By way of example, inset 25 shows catheter 40 as a PENTARAY® mapping catheter (made by Biosense-Webster, Irvine, California), which comprises one or more arms which may be mechanically flexible, each of which being coupled with one or more mapping electrodes. As seen, catheter 40 is fitted at the distal end of a shaft 22.

During the mapping procedure, the mapping electrodes acquire and/or inject signals from and/or to the tissue of left atrium 45. A processor 38 in console 24 receives these signals via an electrical interface 35, and uses information contained in these signals to construct an electro-anatomical map 31. During and/or following the procedure, processor 38 may display electro-anatomical map 31 on a display 26. Typically, processor 38 stores electro-anatomical map 31 in memory 41.

Physician 30 navigates the distal end of a shaft 22 to a target location inside left atrium 45 of heart 26 by manipulating shaft 22 using a manipulator 32 near the proximal end of the catheter and/or deflection from the sheath 23. During the insertion of shaft 22, mapping catheter 40 is maintained in a collapsed configuration by sheath 23. By containing mapping catheter 40 in a collapsed configuration, sheath 23 also serves to minimize vascular trauma along the way to the target location.

During the procedure, a tracking system is used to track the respective locations of the mapping electrodes, such that each of the signals may be associated with the location at which the signal was acquired. For example, the Active Current Location (ACL) system, made by Biosense-Webster (Irvine, California), which is described in US Patent 8,456,182, whose disclosure is incorporated herein by reference, may be used. In the ACL system, a processor estimates the respective locations of the electrodes based on impedances measured between each of the mapping-electrodes, and a plurality of surface-electrodes (not shown) that are coupled to the skin of patient 28. Processor 38 calculates a data-set of estimated locations along one or more paths of catheter 40 inside left atrium 45.

The example illustration shown in Fig. 1 is chosen purely for the sake of conceptual clarity. Other tracking methods can be used, such as ones based on measuring voltage signals, as with the CARTO® 3 system (produced by Biosense Webster). Other types of sensing catheters, such as the Lasso® Catheter (produced by Biosense Webster) may equivalently be employed. In an optional embodiment, processor 38 is further configured to indicate the quality of physical contact between each of the mapping electrodes and an inner surface of left atrium 45 during measurement.

Processor 38 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory.

LEFT ATRIUM SHAPE RECONSTRUCTION FROM SPARSE CATHETER MEASUREMENTS USING NEURAL NETWORKS

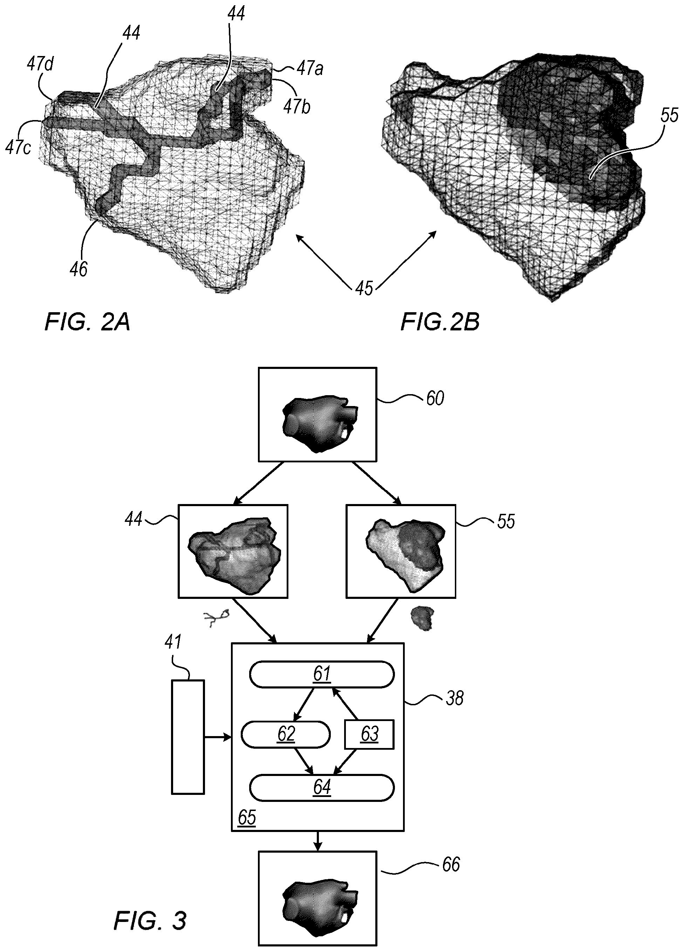

Figs. 2A and 2B are schematic, pictorial illustrations of regions over a left atrium where sets of locations were measured, in accordance with an embodiment of the present invention. Fig. 2A shows an example path 44 along which mapping catheter 40 measured locations, for example, over an inner surface of left atrium 45. Path 44 begins at septum 46 which is the typical entry point of catheter 40 into left atrium 45. The path proceeds to the left superior PV 47a, left inferior PV 47b, right inferior PV 47c and finally to right superior PV 47d.

In additional or alternative embodiments, during a modeling phase, for example, the physician desires an improved representation of the left atrium obtained from electro-anatomical measurements over sparse catheter paths. To provide additional measured locations, a partial surface 55 (i.e., a region) of left atrium 45 is extracted from an imaged partial volume of left atrium 45, such as those acquired by a CT, US, or MRI imaging modality, as exemplified by Fig. 2B. In some embodiments, partial surface 55 is extracted from catheter measurements, such as measurements from multi-electrode catheters such as the PENTARAY® and/or LASSO® mapping catheters (made by Biosense Webster), and/or use a balloon or a basket catheter.

To extract surface 55, the processor typically runs an image processing software, such as software that extracts a partial surface of left atrium 45 from medical images, using the sphere intersection model. In an embodiment, a random sphere is created with a center close enough to the center of the imaged atrium volume to have a reasonable intersection. The processor applies the software to place the selected sphere in intersection with the imaged left atria volume while requiring (e.g., by minimizing the loss function) the network model to reconstruct the full input, i.e., the complete representation of the left atrium.

The example illustrations shown in Figs. 2A and 2B are chosen purely for the sake of conceptual clarity. Figs. 2A and 2B only show parts relevant to embodiments of the present invention. Other details, such as the actual measured locations, are omitted for simplicity of presentation.

Based on the acquired locations, measured in either of the above described techniques, or others known, processor 38 uses the disclosed neural network computation technique to produce a three-dimensional model of left atrium 45, as described below.

In order to reconstruct a realistic volume of left atrium 45 from sparsely measured locations, the disclosed neural network computation technique uses a loss function (i.e., a neural network model), $G$, which includes a regularization-function, $F$, that comprises smoothing spatial weights, in addition to a cross-entropy loss term $L$. In some embodiments, $L$ $(x, z)$ is a logarithmic norm function, based on training, to achieve a "best fit" of $z$ values to the measured locations x:

$$L(x,z) = \sum xlog(z) + (1-x)\log(1-z)$$

where $x$ represents binary occupancy values (i.e., probabilities of being inside an atrium) for every coordinate in the volume of the measured locations, and $z$ represents the binary occupancy values of the reconstructed volume. The summation (not shown explicitly) is performed over all coordinates of $x$, as described, for example, by Vincent et al., in "Extracting and Composing Robust Features with Denoising Autoencoders," Proceedings of the 25th ACM International Conference on Machine Learning, 2008, pages 1096-1103, which is incorporated herein by reference.

The complete neural network model function, G, is then given by:

$$G(x,z) = L(x,z) + F(x,z) = L(x,z) + \lambda \|\nabla_v W(x,z)\|^2$$

The above disclosed minimization of $F(x, z)$ provides a smooth reconstruction, where the level of smoothing is set by a non-negative parameter $\lambda$. The function $W(x, z)$ denotes a term comprising spatial weights, and an example of it is described in the above cited conference paper by P. Vincent et al. In the disclosed technique, $W(x, z)$ is differentiated with respect to spatial dimensions $v$, forming a contractive autoencoder that penalizes non-smooth reconstructions.

In some embodiments of the present invention, the differentiation is performed on weights of an input layer (i.e., on weights of the first layer of the neural-network model). The above disclosed differentiation technique that is applied to the first layer is named hereinafter Weights Smoothing Regularization (WSR). In an embodiment, WSR results is sufficiently smooth 3D representation of a cardiac chamber, and thus, saves a need to include derivative of weights from additional layers in the regularization-function, $F$.

In an embodiment, the above functions are implemented discretely in software, so that, for example, a derivative with respect to $v$ is computed using a finite difference method applied on matrix elements.

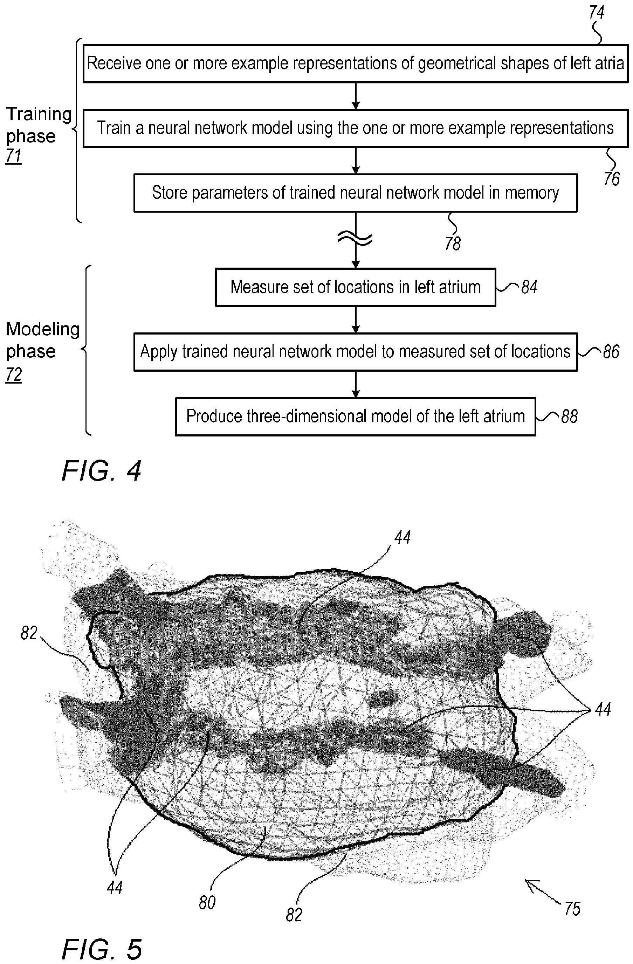

An example of an experimental application of the disclosed technique, using locations measured over a catheter path inside a left atrium laboratory phantom, and the application of $G(x, z)$ to reconstruct the left atrium laboratory phantom, is provided in Fig. 5 below.

The neural network model is brought above by way of example. Other neural network models, e.g., ones that apply other metrics, such a different norm L, are possible, as would occur to a person skilled in the art.

Fig. 3 is a block diagram that schematically illustrates a system for reconstructing a shape of left atrium 45 using a trained neural network model, in accordance with an embodiment of the present invention.

Object 60 is an actual left atrium, such as a laboratory phantom 75 of a left atrium of Fig. 5. The disclosed method is configured to reconstruct object 60 from an input layer 61 comprising sparse location measurements (e.g., paths 44 and/or partial surface 55 of Figs. 2A and 2B, respectively). Input layer 61 and parameters of the trained neural network model are stored in memory 41.

To reconstruct object 60 from sparse data, processor 38 applies an input layer 61 an autoencoder module 65 comprising a neural network module 62 and a regularization module 63. Processor 38 applies autoencoder 65 using a given number of hidden layers of the neural network model, and a given number of voxels to represent input layer 61. Processor 38 generates a 3D output layer 64 that is the reconstructed left atrium 66 (e.g., reconstructed left atrium 80 of Fig. 5) having a same given number of voxels as assigned to input layer 61, but one that comprises a learned left atrium shape that the sparse data best fit into.

Fig. 4 is a flow-chart that schematically illustrates a method for reconstructing a shape of left atrium 45 using neural networks, in accordance with an embodiment of the present invention. The process begins with a training phase 71, after which the disclosed acquisition and modeling systems are operated in a modeling phase 72. The two phases may run at least partially in parallel while preforming a catheterization session.

Training phase 71 begins with processor 38 receiving example representations of geometrical shapes of left atria, at a database uploading step 74. Next, processor 38 runs the disclosed neural network model over the database of example representations, so as to train the network model, at a neural network training step 76. Finally, processor 38 stores the parameters of the trained neural network model in memory 41, or in a disk, or keep the model such way that the model can be directly loaded later during the procedure, at a storing step 78.

In modeling phase 72, electro-anatomical system 20 measures a set of locations in left atrium 45, as described above, in a locations acquisition step 84. Next, processor 38 runs the disclosed trained neural network model from step 76 over the measured set of locations, at a neural network model running step 86. Finally, processor 38 produces a three-dimensional model of left atrium 45, at a neural network modeling step 88.

The process may loop back to step 84 in order to receive more measured locations, for example from another region of heart 26, until the three-dimensional reconstruction is completed.

The example flow-chart shown in Fig. 4 is chosen purely for the sake of conceptual clarity. In alternative embodiments, additional steps may be performed, such as uploading medical images of left atrium 45 from memory 44 and extracting locations from the images, as described above, to provide additional locations for modeling step 86.

Fig. 5 is a schematic, pictorial illustration of a left atrium shape reconstruction 80 of a laboratory phantom 75, in accordance with an embodiment of the present invention. The reconstruction model applies neural networks to a database of locations obtained from sparse catheter path 44. A comparison is made against a ground truth 82, which is the actual shape of left atrium laboratory phantom 75. The set of locations along path 44 was acquired using an electro-anatomical tracking system 20, applied to laboratory phantom 75.

As seen, the acquired location over path 44, combined with a neural network shape reconstruction 80, yields a three-dimensional model of the left-atrium phantom that is substantially complete, and adequately agrees, with the actual shape of left atrium laboratory phantom 75. Thus, reconstructed shape 80, together with acquired locations over path 44, allows a physician to draw, for example, contours over shape 80 which indicate where to ablate a real (e.g., ground truth 82 in the described case) left atrium tissue to suppress an arrhythmia.

The illustration shown in Fig. 5 is brought purely by way of example, for the sake of conceptual clarity. Any actual shape, for example, may be reconstructed by the disclosed technique using additional information on top of that based on electro-anatomical catheter 40 paths, such as on medical images of left atrium 45.

## SPARSE PATH RECONSTRUCTION

A given EAM system uses the locations traversed by the catheter in order to create the anatomy surface. As described in «L. Sciarra, et al., Utility of newly available carto 3 mapping system to guide catheter ablation of atrial fibrillation, in: EUROPEAN HEART JOURNAL, Vol. 31» the efficiency of the CARTO® 3 System fast anatomical mapping (FAM) has been examined in 25 patients undergoing PVI procedures. They used a LASSO® catheter circular multi-electrode catheter to map the LA. Each FAM point was defined as the average of the catheter locations in a one second respiratory gated window. Once enough points were acquired the surface was reconstructed using an alpha shape algorithm. Measurement indicated that mapping with FAM took $9\pm3$ minutes.

The accuracy of the surface was compared to an MRI scan and yielded a mean 138 distance of $3.46\pm0.02$mm. This gave a vein isolation success rate of 96 percent. Note that for many cases the quality of FAM after 10 minutes is not satisfactory; thus often physicians take more than 20 minutes to make the mapping, and furthermore, manual shape-editing steps are required following the mapping.

US 9,576,107 (which is incorporated herein by reference), describes "model FAM (mFam)", a blending of shape models is used with learned statistical modeling of shape parameter distributions. This model initiates with a sparse catheter path (similar to our considered input) and provides LA reconstruction. Later in the procedure, the reconstruction is improved using additional physician inputs and accumulated catheter data. The method shows promising results, however, so far it has been evaluated only by visual inspection of the physicians.

Works can be found in the literature that shows success in reconstructing 3D shapes from partial data using generative networks (see <<E. Ahmed, et al., Deep learning advances on different 3d data representations: A survey, CoRR abs/1808.01462>>).

As described in <<A. Brock, et al., Generative and discriminative voxel modeling with convolutional neural networks, arXiv preprint arXiv:1608.04236>>, the authors generated 3D volumes of different shapes and interpolated them using a variational auto-encoder combined with a fully convolutional neural network. Several works focus on segmenting and reconstructing heart chambers, with either 2D images as input or 3D volumes (see «C. Chen, et al., Deep learning for cardiac image segmentation: A review, Front. Cardiovasc. Med. 7»).

As described in «M. Avendi, et al., A combined deep-learning and deformable-model approach to fully automatic segmentation of the left ventricle in cardiac mri, Med. Image Anal 30 (2016) 108-119», an auto-encoder was used to train a 2D CNN to detect and segment the left ventricle in an MRI image.

Recently, «H. Liao, et al., More knowledge is better: Cross-modality volume completion and 3D+2D segmentation for intracardiac echocardiography contouring, in: Lecture Notes in Computer Science (including subseries Lecture Notes in Artificial Intelligence and Lecture Notes in Bioinformatics), 2018» used several ultrasound views, acquired using an intracardiac catheter, as a 3D input to a V-Net (see «F. Milletari, et al., V-Net: Fully convolutional neural networks for volumetric medical image segmentation, in: Proceedings - 2016 4th International Conference on 3D Vision, 3DV 2016, 2016») that inferred the complete LA shape. The V-Net was trained using GAN. A second network used the complete 3D shape information to improve the LA segmentation of the ultrasound slices.

In «Z. Xiong, et al., Automatic 3d surface reconstruction of the left atrium from clinically mapped point clouds using convolutional neural networks, Front. physiology 13 (2022) 880260-880260, 35574484[pmid]», a V-Net variant is used to reconstruct the LA shape using point clouds acquired by clinical mapping systems. This work is one of the first that combines the network solutions within this task domain. In the approach suggested, 20% to 40% of the atria surface is sampled, to produce the point cloud sample input to the network. Strong dice scores and surface-to-surface distance are presented. In the current work, we are approaching the task of the LA shape reconstruction from a different input perspective: we focus on a rapidly-acquired catheter path that traverses anatomical landmarks only. Most of this path is in the blood-pool and we do not require it to touch any portion of the surface.

This Section describes our framework which generates realistic sparse data from LA shapes and completes the full shape using neural networks. The proposed system teaches a learning algorithm to reconstruct the shape of the left atrium from a catheter path.

Due to the limited number of ground-truth CT shapes with corresponding catheter paths, we simulated this data using the Biosense LA instance generator (see «F. Milletari, et al., V-Net: Fully convolutional neural networks for volumetric medical image segmentation, in: Proceedings - 2016 4th International Conference on 3D Vision, 3DV 2016, 2016») and a catheter path generator to train the model.

Figure 7 shows the data generation phase (left), where we create a sample of LA and a synthetic catheter path inside it. The catheter trajectory is the input to the network which reconstructs the LA surface (right). In the training process, a path, represented by a binary occupancy volume, which would closely resemble the actual path in a typical clinical scenario, is generated using given anatomical landmarks. In the clinical case, a point cloud is acquired by a catheter and then discretized into a volume. The result is a volume where a voxel indicates whether the catheter traversed it as depicted in Figure 7. The atrium instance sampling process is discussed in Section 3.1, The path is created using a graph-based algorithm, as described in Section 3.2. In the reconstruction phase, this path is uploaded to a neural network such as the encoder-decoder network and the V-Net to reconstruct the original LA shape, as explained in Section 3.3 and Section 3.4, respectively.

### 3.1. Input Data Generation: Synthetic Atria

We represent the shape data as a binary 3D volume of size $45^3$ voxels, where each voxel represents a volume of $2.666mm^3$. The volume describes a set where each voxel is assigned the value of one if it is inside or on the boundary of the set, and zero otherwise, namely an occupancy volume.

We use a predefined model for an atrium shape as described in US 9,576,107, which is incorporated herein by reference. An atrium is defined as a blending of parametric tube like shapes that undergo a non-linear transformation to create the atrial shape.

A statistical model based on features such as PV positions, PV orientations, and ridge locations is modeled as a multivariate normal distribution (MVN) whose parameters are learned from CT scans. A generated sample from the model is given a statistical score using the model of how likely it is to represent an atrium.

To generate a synthetic left atria we sampled from the MVN of the model parameters. Then, we keep samples that score well within the statistical model.

Our generated dataset contains 5006 and 1800 samples for training and testing, respectively.

### 3.2. Input Data Generation: Generating Synthetic Paths

Next we describe the algorithm to generate a path inside an input LA shape. The path must closely resemble the actual catheter traversal performed in the clinic. We assume a catheter with a single mapping sensor for this application.

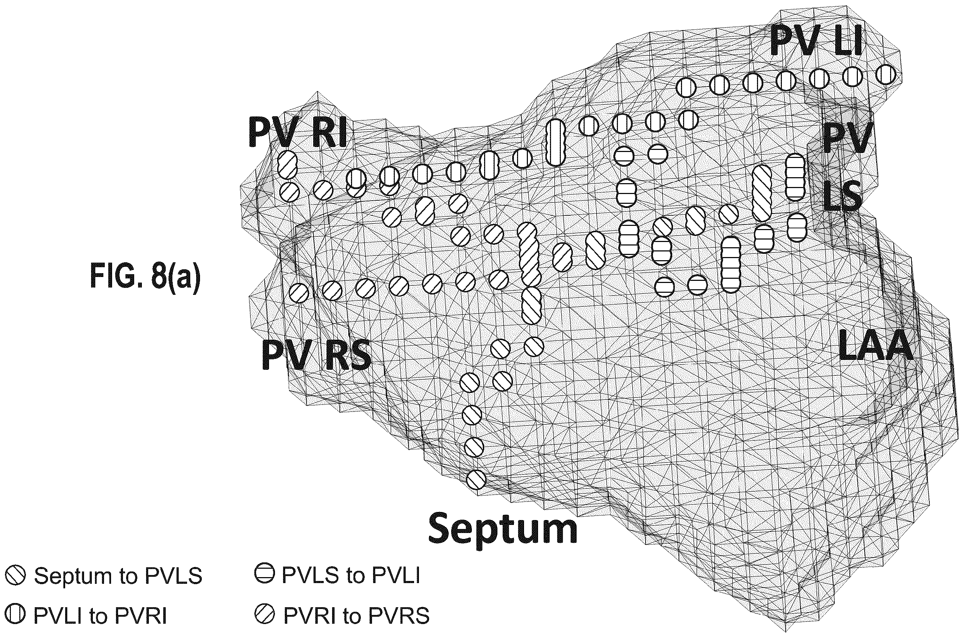

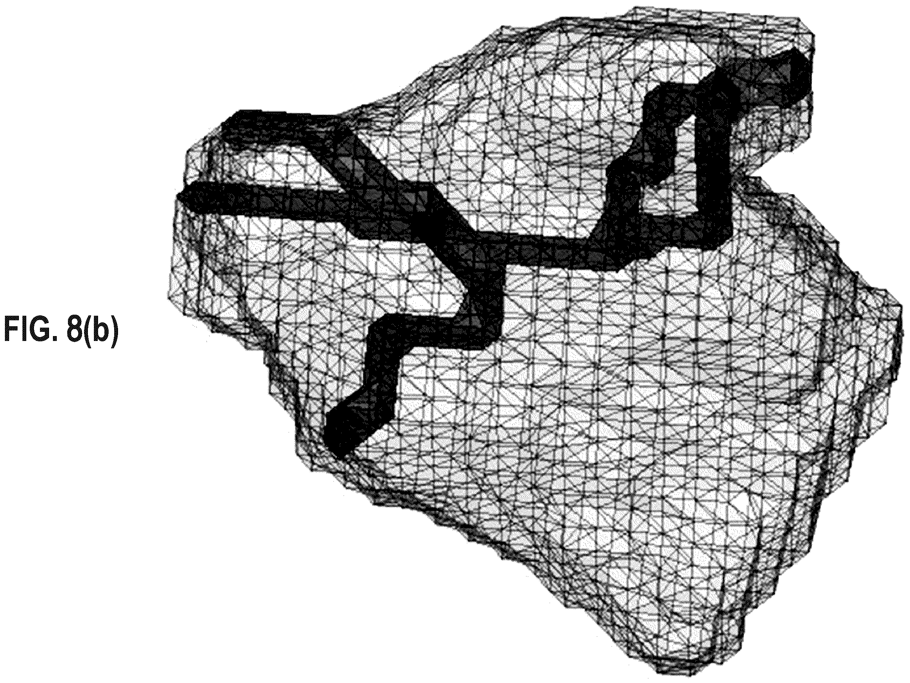

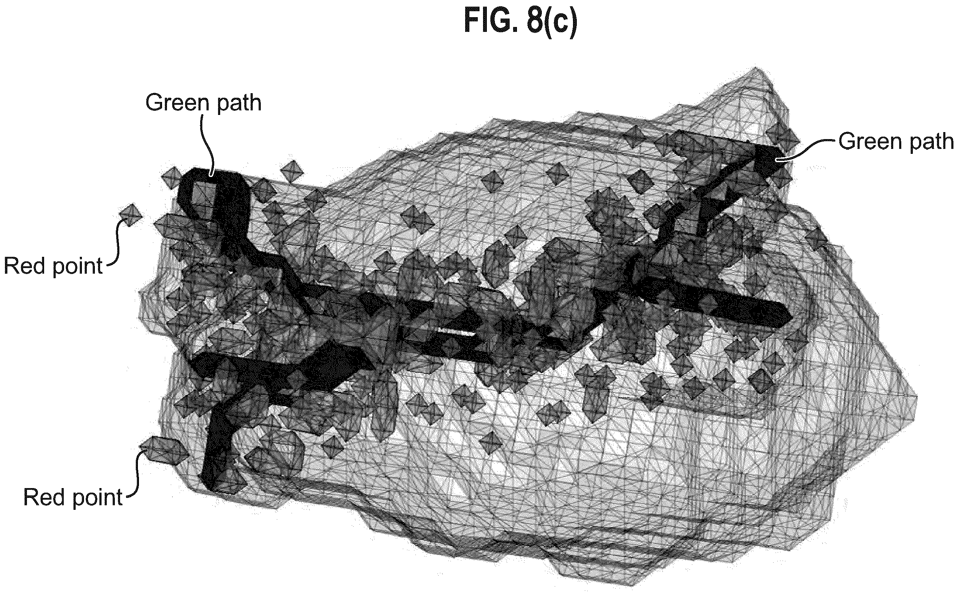

The path is generated in the following sequence: starting at the septum (the entry point from the right to the left atrium), the path proceeds to the left superior, left inferior, right inferior and finally to the right superior PVs. Each such step represents a certain section in the overall path. Figures 8a, 8b, and 8c show different path sections (a) and the overall composed path (b). The proposed algorithm is capable of generating a multitude of simulated paths in the large group of simulated left atria in our generated dataset.

The procedure to create a path is as follows: First, we locate a point in the ostium (entry point) of each PV. Then, applying a predefined traversal order, we find a path from each ostium to the other by solving a graph optimization problem. The graph formulation trades off the shortest path distance with navigational feasibility in the clinic. We solve the graph problem by using the Dijkstra algorithm, as described in «T. H. Cormen, et al., Introduction to Algorithms, Third Edition, 3rd Edition, The MIT Press, 2009».

We next review the main steps in the path creation.

Additional detail is provided in the Appendix.

### 3.2.1. Simulated Paths Creation Procedure Overview

The input LA shape for this algorithm is represented as a triangulated mesh.

The mesh is a collection of vertices and faces (triangular) that describe the surface boundary of the atrium. In a preliminary stage, an Atria mean shape was generated by taking a voxel-wise average over all the generated LA used for training. We manually chose the landmark coordinates of the mean shape which are the four ostia points (at the center of the ostium) and approximate septal point. For each new input, we located the PVs' ostia points and the Septum.

For this, the mean shape landmark points were projected to the current sample via the algorithm described in Appendix A. 1. The input is then converted into a voxel-based representation by sampling the points of the coordinate grid that are inside the mesh surface (assigned a value of one) or outside (zero valued).

The sampling resolution for the path generation is $2.666mm^3$ per voxel in a grid of $45^3$ voxels. In total, this covers a volume of $12cm^3$, which contains most atria. We define a trajectory as a sequence of adjacent voxels originating in the septum through the four PVs' ostia points. Due to the small number of degrees of freedom of the catheter, the trajectory seldom follows a straight line. A realistic path tends toward the center of the atrium before reaching the next target point. Appendix A.2 describes this formulation. As an outcome of the procedure above, the algorithm returns a volume where marked voxels (with value one) lie on the path, and zero otherwise.

### 3.2.2. Synthetic Path Augmentation

The generated synthetic path was augmented by adding nearby points which are mostly inside the corresponding atrium. The augmentation procedure is motivated by the catheter's physical setting and was tested empirically. The catheter vibrates during movement and may exceed the chamber boundary by slightly pushing it. First, for each (grid sampled) path point $x_p$ we sample n points normally distributed around it, $x_n \sim N(x_p, \sigma)$. Then the points are trimmed using a probability factor $s_f$, for each point. Next, we only consider points that are interior to the ground truth mesh. These undergo a normally distributed translation $t_f \sim N(0, 1) \times \mu_s$, where $\mu_s$ is the factor that determines the noise level. Figure 8c shows an augmentation result.

### 3.3. Left Atrial Shape Reconstruction using DED Network

We next focus on the reconstruction part of this work. We propose an NN-based reconstruction of the complete LA shape from a given sparse catheter path. A given network is trained on synthetic paths. The output of the network is a probability volume, resulting from a sigmoid function applied in the last layer. This layer represents the probability of each voxel being the interior (or on the boundary) of the atrium. The value of each voxel is converted to a binary value by setting a 0.5 threshold.

In this study, we chose the dense-encoder-decoder (DED) as our model of choice. This architecture is based on the auto-encoder (AE), which is a neural network that performs non-linear dimensionality reduction, similar to the non linear (kernel) principal component analysis (PCA). A binary vector input $x \in \{0, 1\}^s$ is mapped to a hidden representation $y \in [0, 1]^d, d \ll s$. A deterministic mapping $y = \sigma(Wx + b)$, is used, where $W : s \to d$ is an $[d \times s]$ matrix and $b \in R^d$ and $\sigma$ is a non-linear squashing function such as a sigmoid or a tanh. A decoding layer is then used to reconstruct the input using the following transformation: $\bar{x} = \bar{\sigma}(\bar{W}y + \bar{b})$ where $\bar{W} : d \to s$ is a matrix and $\bar{b} \in R^s$.

Our model used 'tied weights' $\bar{W} = W^t$, and masked input (as described in <<P. Vincent, et al., Extracting and composing robust features with denoising autoencoders, in: Proceedings of the 25th international conference on Machine learning, ACM, 2008, pp. 1096-1103>>), which is equivalent to a dropout layer after the input. This was found experimentally to give the best outcomes (without it, the results degraded significantly). The Adam optimizer (as described in «D. P. Kingma, J. Ba, Adam: A method for stochastic optimization (2014)») was used. All the layers except the last used RELU activation.

The last layer used the sigmoid activation function $\sigma(x) = 1/(1+e^{-x})$. As described in <<S. Ioffe, C. Szegedy, Batch normalization: Accelerating deep network training by reducing internal covariate shift, in: Proceedings of the 32nd International Conference on International Conference on Machine Learning - Volume 37, ICML' 15, JMLR.org, 2015, p. 448-456>>, batch normalization layers can be used following each layer (except the last) to normalize the activation in the network making it more robust to the difference between the synthetic and clinical paths. Figure 7 provides an illustration of the network.

The network was trained using a linear (convex) combination, L(x, z), of the cross entropy loss (as described in <<P. Vincent, et al., Extracting and composing robust features with denoising autoencoders, in: Proceedings of the 25th international conference on Machine learning, ACM, 2008, pp. 1096-1103>>) and the negative of the DICE coefficient (as described in «F. Milletari, et al., V-Net: Fully convolutional neural networks for volumetric medical image segmentation, in: Proceedings - 2016 4th International Conference on 3D Vision, 3DV 2016, 2016»).

For a training sample z the cross entropy loss is defined as

$$CE(x, z) = \sum_{i=1}^{n} \hat{x}_i \log z_i + (1 - \hat{x}_i) \log(1 - z_i)$$

where summation is over all voxels. The weighted DICE is defined as:

$$WDICE(x, y, w_v) = \frac{2 w_v x^T w_v y}{w_v x^T w_v x + w_v y^T w_v y}$$

Note that the non-weighted DICE is obtained by setting all weights to one.

*Spatial Weight Smoothing Regularization (SWR)*

In order to reconstruct a realistic atrial volume, we added a Spatial Weight Smoothing Regularization (SWR) term to the loss function. The loss including SWR is defined as:

$$L(x, z) + \lambda \sum_{i=1}^{n} \| \nabla_v W_i \|^2$$

where W denotes the layer weights and the differentiation is with respect to the spatial dimension $\vec{v}$, that is the position within the volume (for the three spatial axes, $\vec{v} \in (i, j, k)$) in which the input and output reside. $\lambda$ represents the level of regularization.

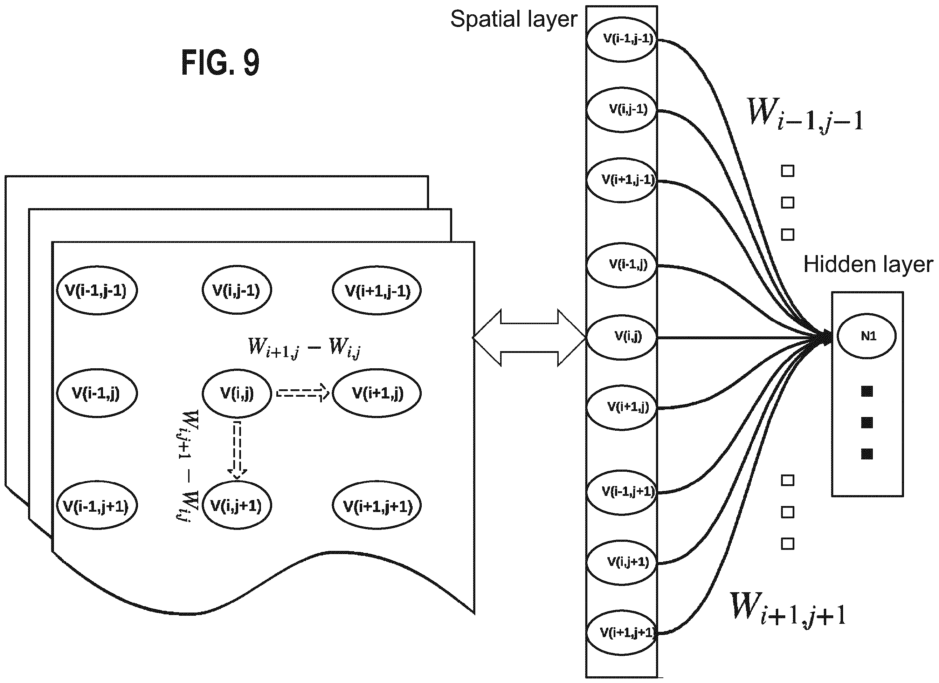

The SWR loss term is applied to the weights of the input and output layers only, for which each weight corresponds to a voxel. The spatial derivatives are computed using finite differences. Figure 9 depicts the relationship between the voxels in space and the neurons in the layer and shows which weight difference is added to the cost.

The goal in adding the SWR term is to have the weights of the first layer that convert volumetric input to output and vice versa (same for the case of tied weights) be a smooth function in $R^3$, as are heart chambers. This obviates the need to use smoothing as a post-processing step. We used a similar concept to the active contours approach (as described in «M. Kass, A. Witkin, D. Terzopoulos, Snakes: Active contour models, Int. journal computer vision 1 (4) (1988) 321-331») where the external energy is the cross entropy loss and the internal energy is SWR. It is also similar to the interpretation of eigenvectors in PCA. We assume our model learns a blend of shapes that are smooth in nature; each resembles a complete or part of an atrium. The task of the model at inference is to compute which parts to take in order to compute the most probable atrium given the input. As a regularization term, this cost helps to prevent over-fitting.

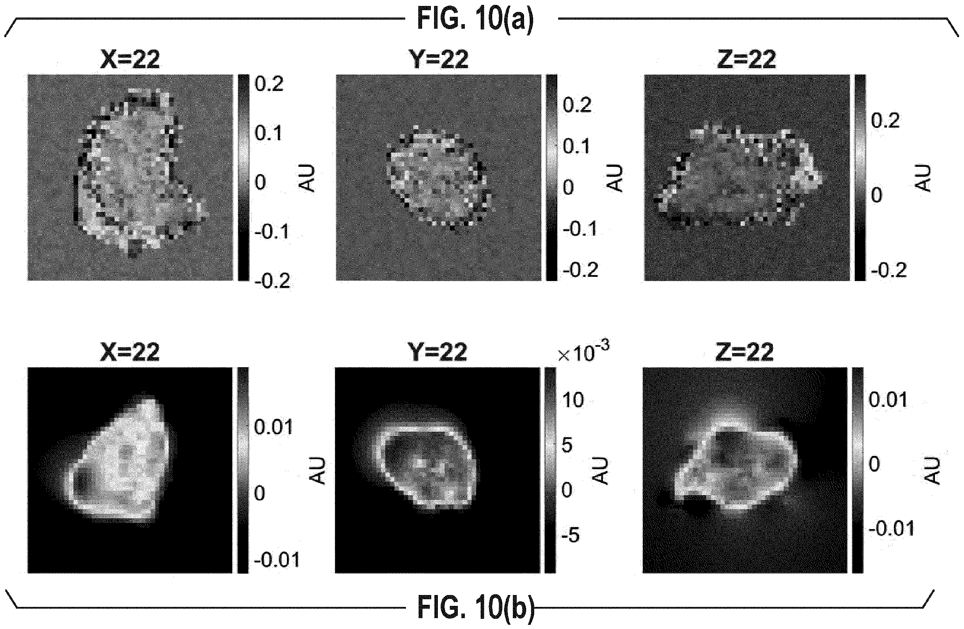

The effect of the SWR can be seen in Figure 10. We examine a weight from the first layer of two similar networks, one trained with SWR while the other without. The selected weight is reshaped to a $45^3$ voxel volume, maintaining the spatial ordering. In Figure 10 we take slices through the three axes of this volume and show these as images. While the network without the SWR learns atria parts with fuzzy boundary, see Figure 10, it is evident that the network with SWR in Figure 10 learns smooth parts that seem like atria building blocks.

*Boundary Enhancement Mask*

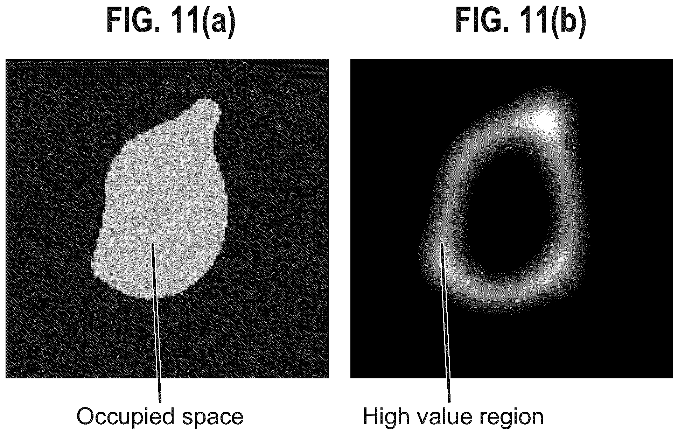

The most significant area in the volume is the surface boundary. In order for the model to have larger loss gradients around the surface boundary, we used the weighted DICE cost with a weighting mask W. The mask assigns a weight for each voxel such that the majority falls over the boundary, and decreases for voxels further away from it, as seen in Figure 11. The weight of a voxel v is given by:

$$W = (1 + \alpha)/(1 + PN(D(v)))$$

, where D(v) is the distance from the shape boundary and PN is the probability density function of the normal distribution with zero mean and $\sigma$ = 1.5. The $\alpha$ parameter was experimentally set to 14.

*DED Parameter Selection*

Subsequent to empirical experimentation, we report the results of the best performing DED variant. During our experimentation, we tested different combinations of depths and widths (in accordance with <<A. Baram, et al., Left atria reconstruction from a series of sparse catheter paths using neural networks, in: F. Knoll, A. Maier, D. Rueckert (Eds.), Machine Learning for Medical Image Reconstruction - First International Workshop, MLMIR 2018, Held in Conjunction with MICCAI 2018, Proceedings, Lecture Notes in Computer Science (including subseries Lecture Notes in Artificial Intelligence and Lecture Notes in Bioinformatics), Springer Verlag, 2018, pp. 138-146>>).
The chosen variant includes two hidden layers with 350 neurons each. The cross-entropy and (weighted) DICE were combined in the loss function using a ratio of 2:3. The variants are named according to the SWR parameter $\lambda$: "No SWR" with $\lambda$= 0, "SWR005" $\lambda$= 0.05, "SWR75" $\lambda$= 75. "No Aug" net has the same parameters as "SWR005" but with no path augmentation and no boundary enhancement mask.

3.4. Left Atrial Shape Reconstruction using V-Net Network

The recent success of convolutional neural networks (CNNs) for vision-related tasks motivated us to conduct experimentation with these networks. Fully convolutional networks provide a classification result for each pixel: The U-Net (see <<O. Ronneberger, et al., U-net: Convolutional networks for biomedical image segmentation, in: Lecture Notes in Computer Science (including subseries Lecture Notes in Artificial Intelligence and Lecture Notes in Bioinformatics), 2015>>) for 2D images and the V-Net (see «F. Milletari, et al., V-Net: Fully convolutional neural networks for volumetric medical image segmentation, in: Proceedings - 2016 4th International Conference on 3D Vision, 3DV 2016, 2016») for 3D volumes have shown promising results in many biomedical image processing applications such as breast tissue segmentation in MRI (see «M. U. Dalmi,s, et al., Using deep learning to segment breast and fibroglandular tissue in MRI volumes:, Med. Phys.arXiv:0608246v3») and pancreatic tumor segmentation in CT (see «Z. Quo, et al., Deep LOGISMOS: Deep learning graph-based 3D segmentation of pancreatic tumors on CT scans, in: Proceedings - International Symposium on Biomedical Imaging, 2018»).
Recall that the DED output, similar to the V-Net, provides a classification label for each voxel in the volume to be either inside or outside the atrium.
The V-Net is a combination of several convolution layers followed by max pooling, each shrinking the volume by half, after each stage. This is repeated for four stages. The next steps consist of up-sampling using learned filters performed over the data in four stages, where residual connections connect the input with information coming from an earlier stage of the same size using concatenation. Batch normalization and dropout are performed to keep the model regularized.
Figure 7 depicts the architecture of the network and the integration with our system. Similarly to the DED the input is the voxelized catheter path while the output is the LA reconstruction, both represented as occupancy volumes. In addition, network parameters such as the number of filters per layer, the number of down/upsampling layers, and concatenation, are depicted. The first and last layers have filters with a larger receptive field, which was found to be critical to the success of the network to provide meaningful results.

3.5. Mean Shape as a Baseline Solution

To determine the effectiveness of any reconstruction algorithm, it needs to be compared to a common solution. In our case, we compared our results to the results of the mean shape solution. The mean shape was generated by taking a voxel-wise average over all the ground truth shapes in the training set. The mean location of a PV ostia over all the available atria data should converge to that of the mean shape. We defined a base coordinate frame for the reconstruction using the mean shape four PV ostia points. The input paths from the clinical cases were registered and transformed to have their PVs ostia match that of the mean shape (in the least squares sense) using rigid point set registration (see «A. Myronenko, X. Song, Point set registration: Coherent point drift, IEEE Trans. on Pattern Anal. Mach. Intell. 32 (12) (2010) 2262-2275»).

## 4. Experiments and Results

We next report the experiments and results in the reconstruction of the left atrial shape. We start with Synthetic path experimentation in Section 4.1 and continue with experiments using human clinical cases, in Section 4.2.

## 4.1. Experiments over Synthetic Paths

### 4.1.1. Dataset

These experiments were conducted over 1800 pairs of paths and the corresponding atria. First, a set of 1800 LA shapes was created using the LA shape generation described in Section 3.1. Then, for each LA, a synthetic catheter path was generated using the process described in Section 3.2.

### 4.1.2. Evaluation Metrics

To evaluate the results of the networks, given a ground truth shape, we use two metrics: The first is DICE, which examines the similarity between the resulting volume to the ground truth. The second metric examines the resulting boundary; It was defined as the set of voxels that separate the interior of the chamber from the exterior. To compare the generated and the true boundaries, we used the average of the distances between all pairs of closest points of the two boundary contours (see, e.g. «K. H. Cha, et al., Urinary bladder segmentation in CT urography using deep-learning convolutional neural network and level sets, Med. Phys. 43 (4) (2016) 1882-1896»):

$$AVDist(\partial \mathrm{x}, \partial \mathrm{y}) = 0.5 \frac{\sum_{u \in \partial \mathrm{x}} min\{d(u,v): v \in \partial y\}}{\|\partial \mathrm{x}\|} + 0.5 \frac{\sum_{u \in \partial y} min\{d(u,v): v \in \partial \mathrm{x}\}}{\|\partial y\|}$$

where d(a, b) is the Euclidean distance between voxels a and b.

### 4.1.3. Results of LA Reconstruction from Synthetic Catheter Paths

We ran the set of networks: 'no SWR', 'NO AUG', 'SWR005', 'SWR75', and V-Net to produce synthetic data results, i.e. LA reconstructions over the synthesized paths of the test set. Table 1 (a) details the results of the described networks and compares these to the mean-shape solution. Note that for DICE the closer to one the better, while for AVdist the lower the better.

Evident from the results shown, all the networks were better than the mean shape by approximately 0.7 - 1mm average distance and 0.05 DICE score. For the DED networks, the networks with SWR outperformed those without. We observe a slight advantage for the 'SWR75' over the rest of the DED networks.

In this testing scenario, the V-Net results were strong. The run time for our DED networks with two layers over moderate consumer-grade hardware (Nvidia GTX2080) was less than 0.9ms for 20 samples.

## 4.2. Experiments in Human Clinical Cases

The networks with the best-performing parameter sets over the synthetic data were used to examine the feasibility of the proposed methods on actual human clinical cases.

**Table 1:** LA Reconstruction from: (a) synthetic paths inputs and (b) clinical cases, comparing DED, V-Net, and Mean shape results.

| | | | Dense Encoder Decoder (DED) | | | | Vnet | Mean Shape |
|---|---|---|---|---|---|---|---|---|
| | | | No Aug | No SWR | 0.05 SWR | 75 SWR | | |
| (a) | | DICE | *0.949* | 0.942 | 0.946 | 0.949 | **0.956** | 0.895 |
| | | AVDist(mm) | 1.331 | 1.45 | 1.388 | *1.32* | **1.204** | 2.261 |

(continued)

| | | Dense Encoder Decoder (DED) | | | | Vnet | Mean Shape |
|---|---|---|---|---|---|---|---|
| | | No Aug | No SWR | 0.05 SWR | 75 SWR | | |
| (b) | Mean Distance (mm) | 5.082 | N/A | 5.137 | **5.015** | 5.256 | 5.79 |
| | Standard Deviation | 1.782 | N/A | 1.863 | 1.058 | 1.032 | 2.995 |
| | p-value (<) | 0.076 | N/A | **0.047** | 0.124 | 0.249 | N/A |

4.2.1. Data Acquisition and Network Input Generation

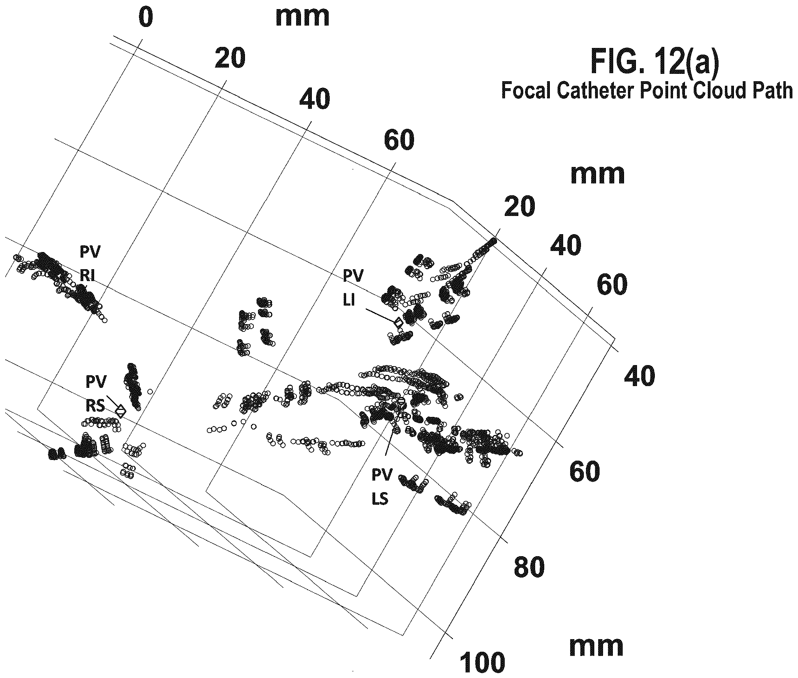

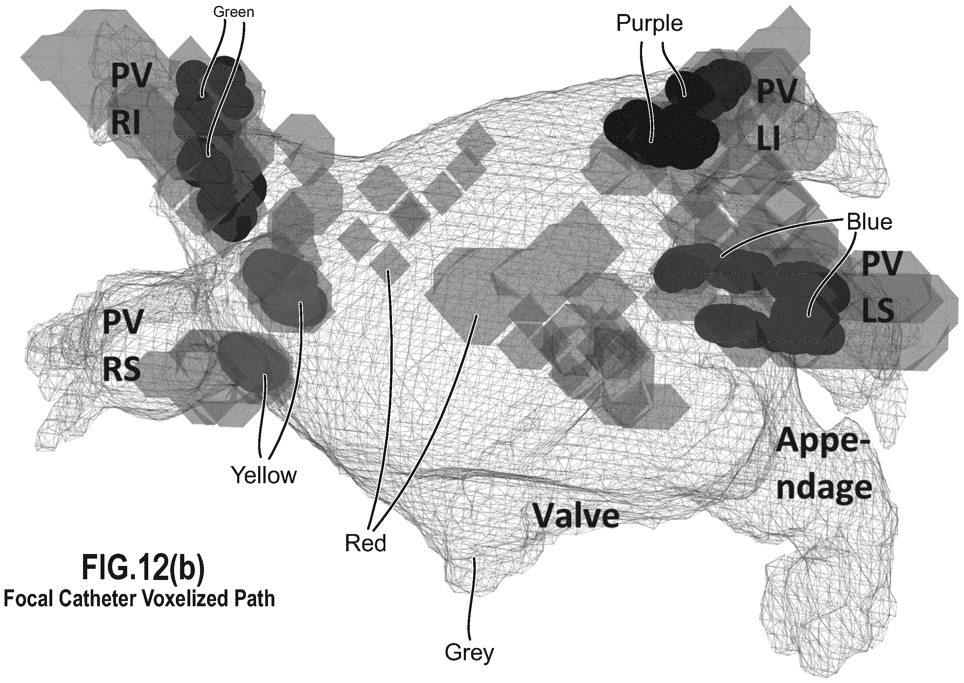

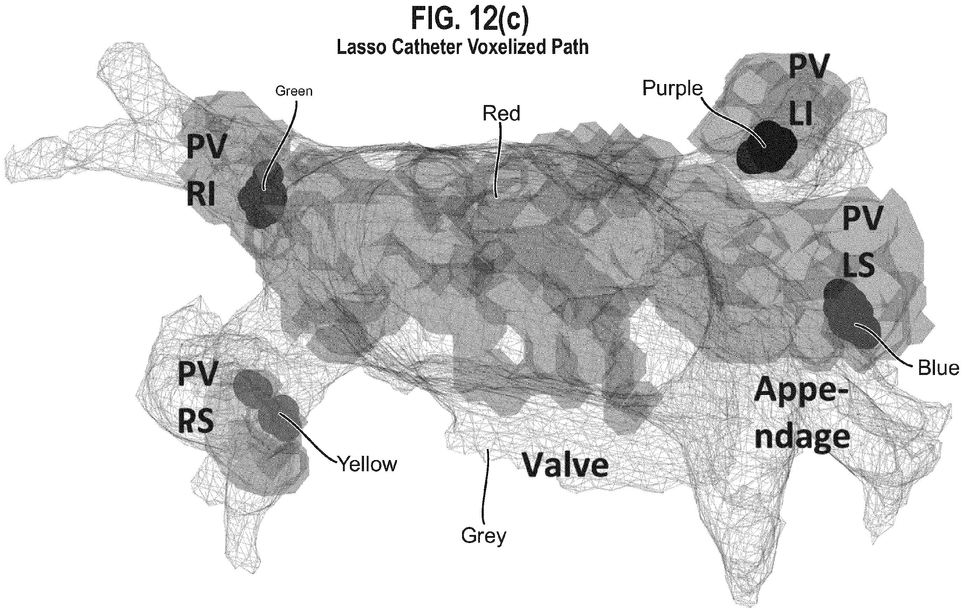

Our clinical dataset was composed of 80 cases, for which different catheters were used. Twenty-six of the cases had a properly registered mesh resulting from a CT segmentation. At the beginning of a clinical procedure, the initial bearing path that starts from the septum, and connects the PVs was acquired, with an acquisition time of fewer than three minutes. The physician tagged the points belonging to each PV ostia. Figure 12a illustrates the input point cloud (path) with tagged PVs centroids. Figures 12b and 12c show the input volumes in red, with colored tagged points for each PV (PVRS yellow, PVRI green, PVLI purple, PVLS blue) and the registered CT. Note that though the path performed in the clinic traversed similar locations, it differed greatly from the generated synthetic paths. Moreover, when the catheter exceeded a predefined velocity, position acquisition is suspended. This created discontinuities in the acquired path. Other differences can be attributed to deviations in the declared protocol from our definitions (visiting other locations, not traversing all locations for our path), the use of multiple arm catheters, different catheter maneuvers, and soon.

The input to the network was generated by implementing the following steps.

First, we need to perform coordinate systems alignment between the acquired path coordinate system and the coordinate system of the networks (which corresponds to that of the mean shape, see Section 3.5). We find each PV ostia of the acquired point cloud by taking the mean of respective PV tagged points.

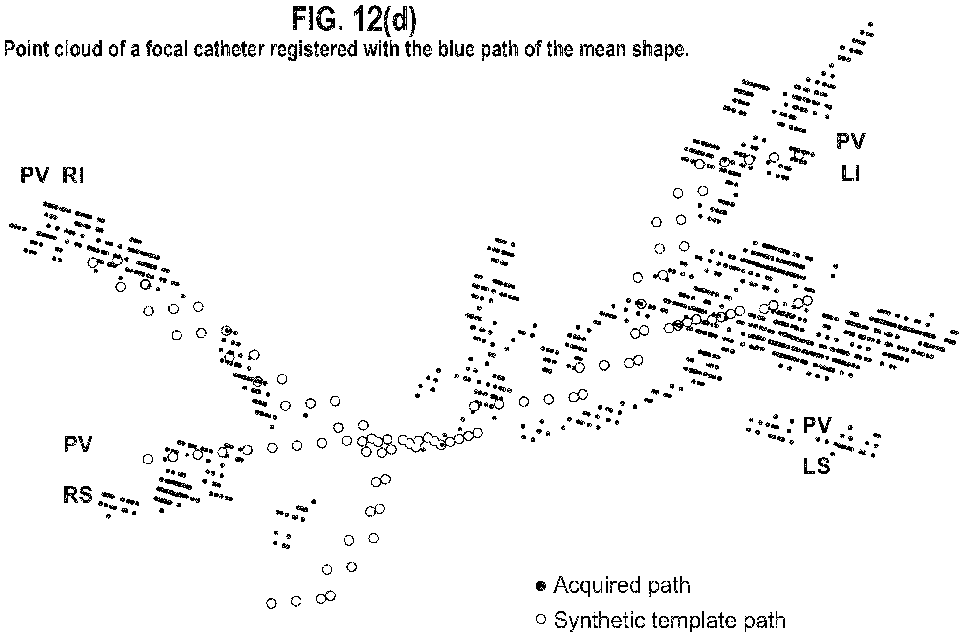

To find the transformation between the two coordinate systems, we applied a rigid point set registration (as described in «A. Myronenko, X. Song, Point set registration: Coherent point drift, IEEE Trans. on Pattern Anal. Mach. Intell. 32 (12) (2010) 2262-2275») matching the PV ostia of the acquired point cloud to the four PV ostia points of the mean shape, see Figure 12d.

This transformation is then applied to the acquired point cloud. In the following step, we convert the transformed acquired point cloud to an occupancy volume. We sampled the point cloud in a 453 voxel volume where each voxel indicated the presence(yes/no) of a point (or several) of the input cloud within the voxel ($2.666mm^3$ per voxel). This volume was the expected input to our network. We note that the input point cloud may differ with catheter type. Figure 12b depicts the input volume for a focal catheter while Figure 12c shows a volume resulting from using a round LASSO® Catheter, where rings of voxels are acquired together.

The network output volume was converted to a triangular mesh by using the Marching cubes algorithm as described in «W. E. Lorensen, H. E. Cline, Marching cubes: A high resolution 3d surface construction algorithm, SIGGRAPH Comput. Graph. 21 (4) (1987) 163-169» implemented in Matlab.

The mesh was defined as the iso-surface at a value of 0.5 of the output volume. We analyzed the performance of the reconstructions using two evaluation methods, the first using contact points while the second compares to ground truth CTs.

4.2.2. Evaluation by Contact Points

Some of the cases used a force sensing catheter (THERMOCOOL SMART TOUCH® SF Catheter) to acquire points that lay on the surface; namely, contact points with a contact force between 5g and 15g, when respiratory gated (end-expirium). These are of interest to physicians since their position is accurate (less than 1mm error) and are usually located near ablation regions and important anatomical landmarks. For this evaluation, we chose cases that use a focal catheter. We included cases in which the acquired point cloud covered most of the synthetic path parts. In addition, there had to be enough ground truth points located in areas of interest such as the PVs. A total of nine cases were suitable.

The reconstruction accuracy was measured as the mean of the distances of the ground truth points from the reconstructed shape. In particular, we measured the distances from each ground truth point to the nearest mesh vertex.

We present the results for the three networks defined in Section 3.3, the VNet, and the mean shape. The reconstruction accuracy results are compared in Table 1 (b). It shows that the errors were highly comparable, although V-Net lagged behind. All the results were highly better than the mean shape; however, due to the variance, the differences in means were significant only for 'SWR005' (paired t-test, pvalue < 0.05), while improving the mean by 0.84mm. We omit the results of DED without SWR since it produced invalid results for some of the samples (jagged disconnected results with no interpretable boundaries).

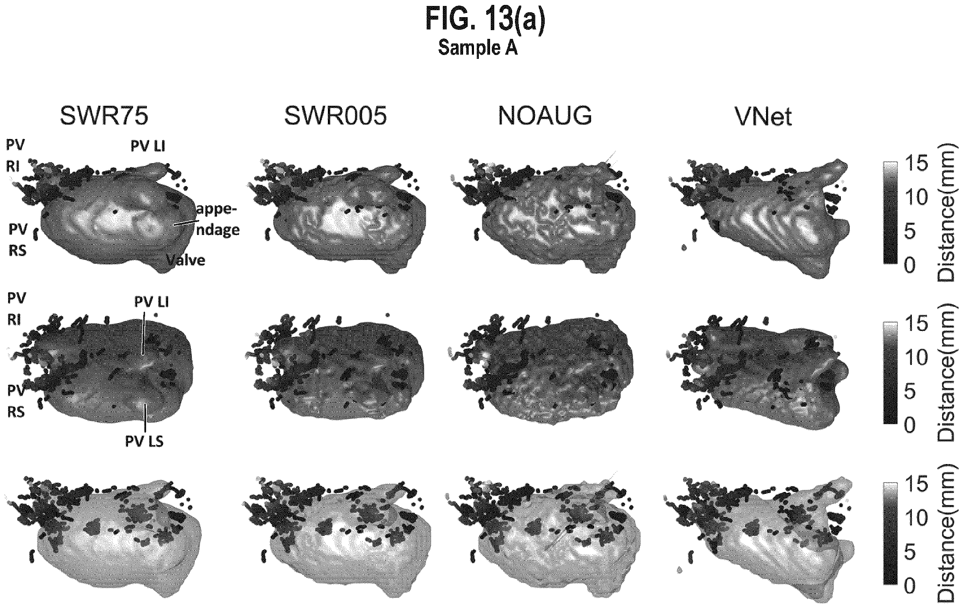

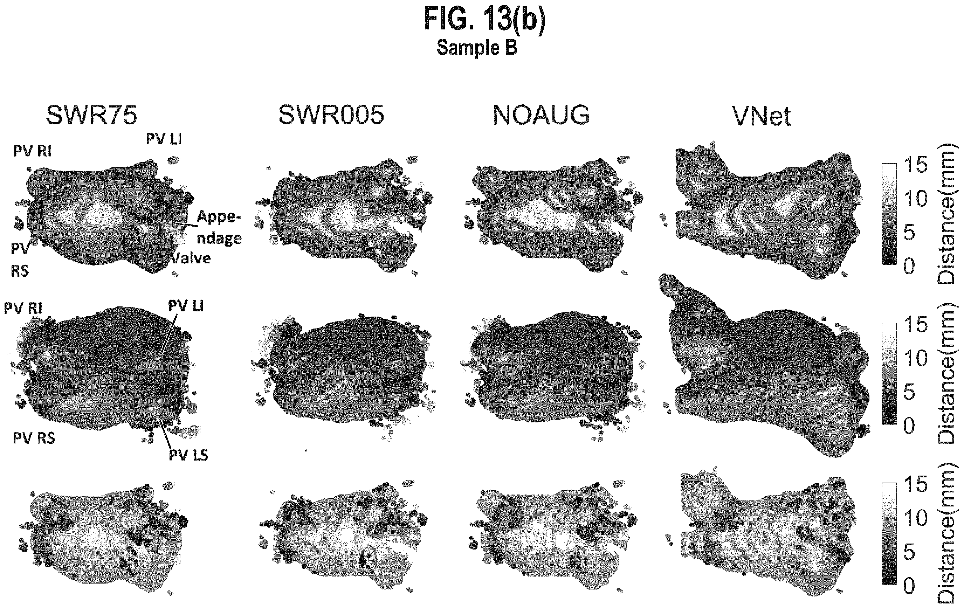

Figure 13 show the resulting surfaces of the tested networks for two cases, overlaid with ground truth points colored by distance to reconstruction. Two views are shown, while the third is a transparent view showing all the ground truth points, colored by distance to the reconstruction. 'SWR005' reconstructions appear smoother than 'NOAUG' reconstructions with more apparent anatomy, thus only 'SWR005' is kept for the next evaluation. ' SWR75' produced better smooth-looking shapes at the expense of making the ridge between the left PVs and the appendage less distinguishable.

### 4.2.3. Evaluation with Ground Truth CT

We gathered a total of 26 cases with a CT that was properly registered to the acquired point cloud representing the catheter traversed location. This enabled us to use the same coordinate system alignment between the point cloud, and the network coordinate system found earlier to bring the CT to align with the network reconstruction result. The cases were obtained from three care centers.

Gender information, provided by one of the centers, indicated even distribution.

Next, we compared the extracted CT surfaces to the reconstructed mesh using the surface to surface distances. Since this metric is largely affected by the surface area of the measurement which is irrelevant in large areas of the LA body we focus on clinically relevant regions. This was done by considering the surface to surface distance within some radius of the physician-tagged points. In this evaluation, the surface to surface distance was computed as follows: Given a vertex on the first mesh, we took the nearest vertex on the second mesh and kept the distance. The total distance is the average overall examined vertices.

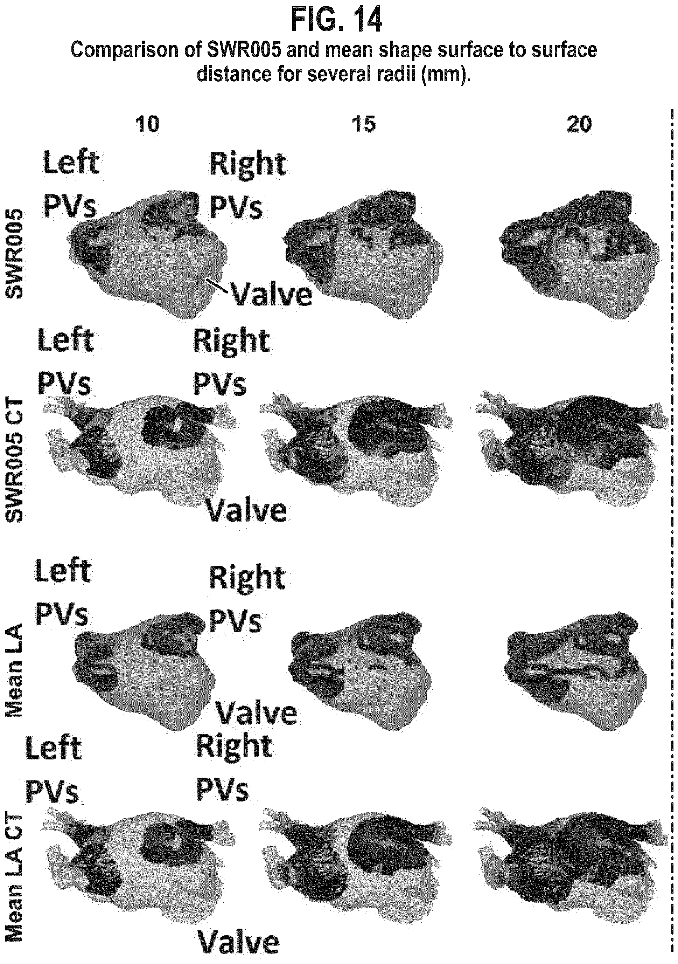

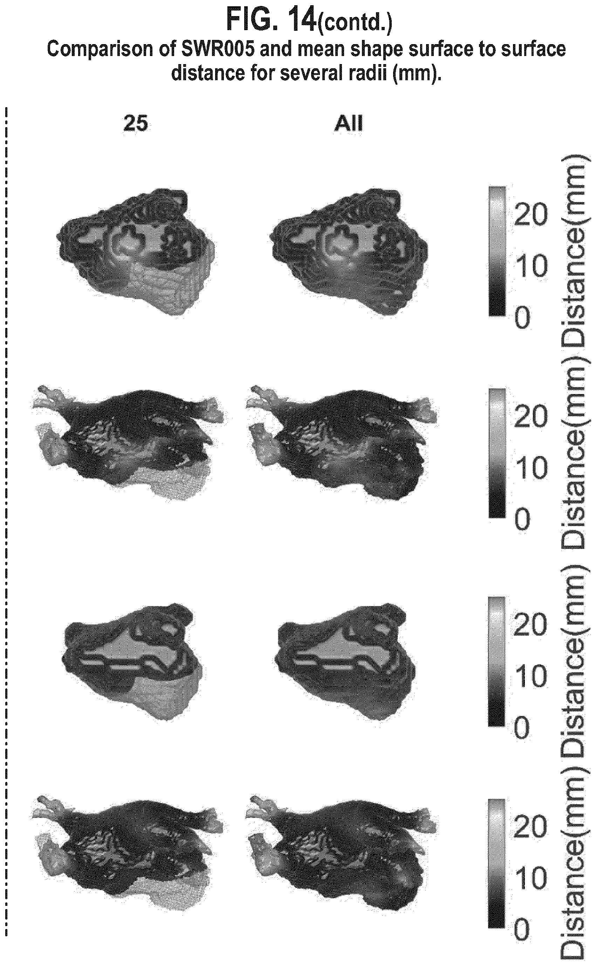

For symmetry, we averaged both the distance from the reconstruction to the CT and vice versa. To examine regions important for the clinic, we only took vertices that were within defined radios of tagged PV points. Figure 14 illustrates the measured regions for possible choice of radii. It is evident that the radius of 15mm captures the PVs ostia surroundings well.

The quantitative results for all 26 CT cases, comparing the surface-to-surface distances for 'SWR005', 'SWR75', V-Net, and the mean shape in various radii are summarized in Table 2, for all cases. The reported p-value indicates the significance of improvement vs. the mean shape (paired t-test, one-tailed).

Quantitatively, 'SWR75', 'SWR005' and V-Net improved the distance metric by 0.3-0.45mm (statistically significant) over the mean shape results for radii between 15 - 25mm.

**Table 2:** Surface to surface distances (mean and standard deviation) comparing LA reconstructions to ground truth CT over 26 clinical cases. Results are shown for DED, V-Net, and Mean shape, in four different radii, and unbounded distance. The p-value tests for significant differences between the network and the mean shape. P-value under the significance level of 0.05 is in italics.

| Distance from Interest Points | | Unbounded | 10mm | 15mm | 20mm | 25mm |
|---|---|---|---|---|---|---|
| **DED 0.05 SWR** | **Mean** | 6.057 | **4.307** | 4.697 | 5.083 | 5.363 |
| | **Std** | 0.858 | 0.888 | 0.0832 | 0.868 | 0.921 |
| | **p-value (<)** | 0.055 | *0.00054* | *0.00039* | *0.00058* | *0.0023* |
| **DED 75 SWR** | **Mean** | 6.149 | 4.327 | **4.678** | 5.093 | 5.357 |
| | **Std** | 0.979 | 0.803 | 0.712 | 0.767 | 0.837 |
| | **p-value (<)** | 0.24 | *0.0091* | *0.0045* | *0.009* | *0.016* |
| **VNET** | **Mean** | **5.962** | 4.441 | **4.678** | **4.984** | **5.216** |
| | **Std** | 0.913 | 0.75 | 0.708 | 0.651 | 0.662 |
| | **p-value (<)** | 0.068 | 0.14 | *0.045* | *0.025* | *0.022* |
| **Mean Atrium** | **Mean** | 6.241 | 4.735 | 5.103 | 5.458 | 5.686 |
| | **Std** | 1.225 | 1.311 | 1.195 | 1.19 | 1.244 |

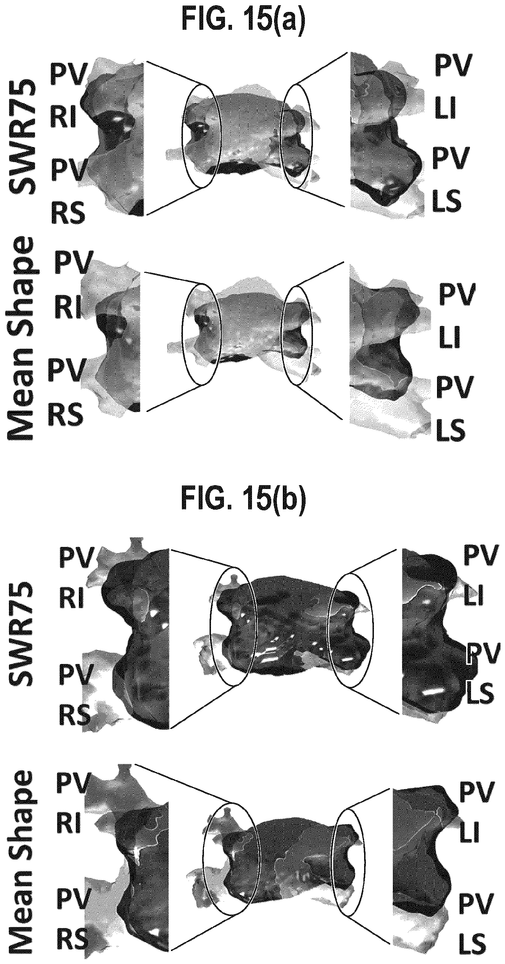

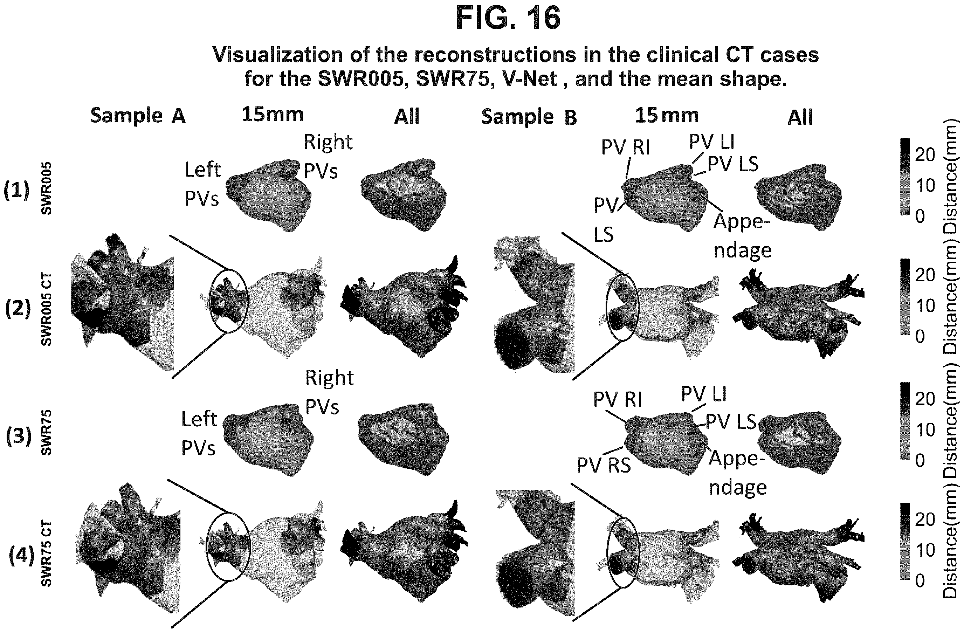

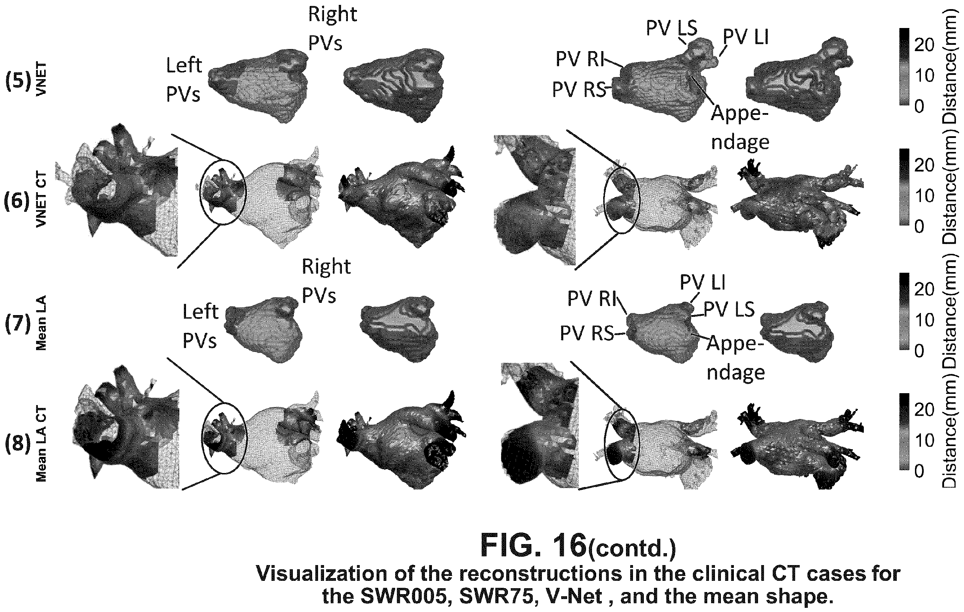

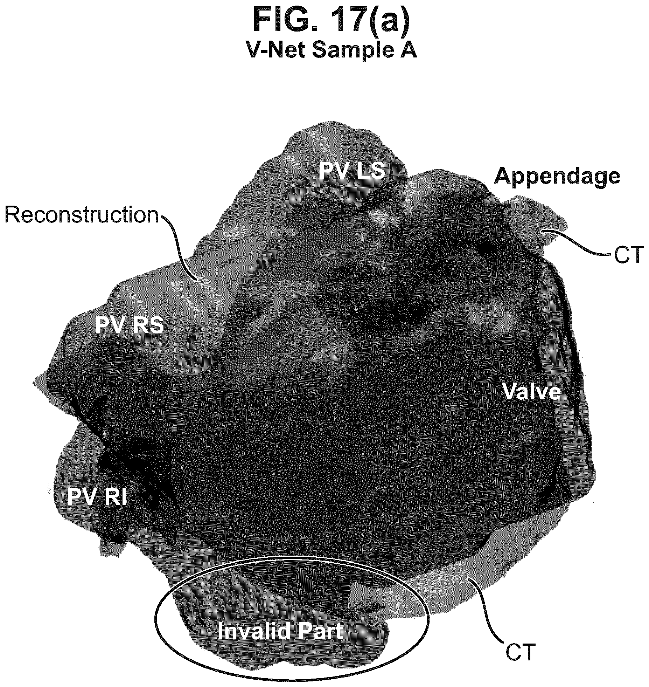

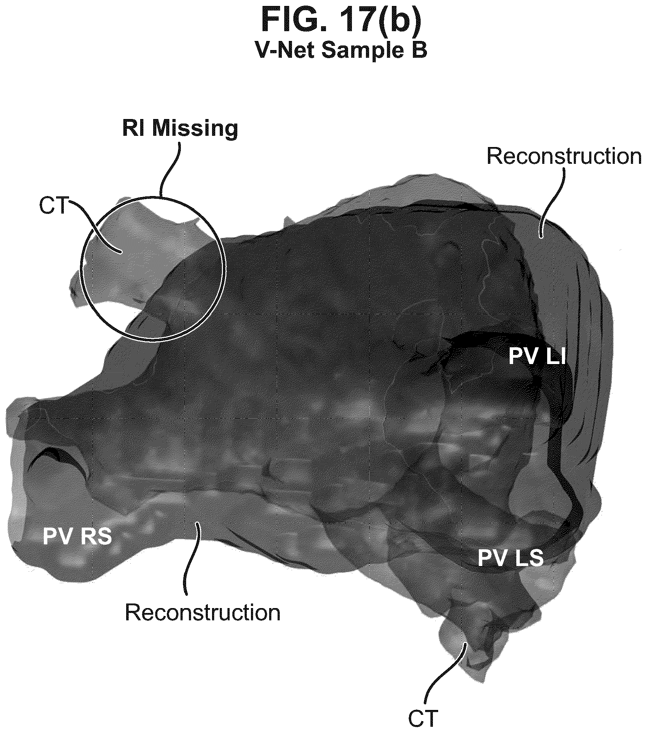

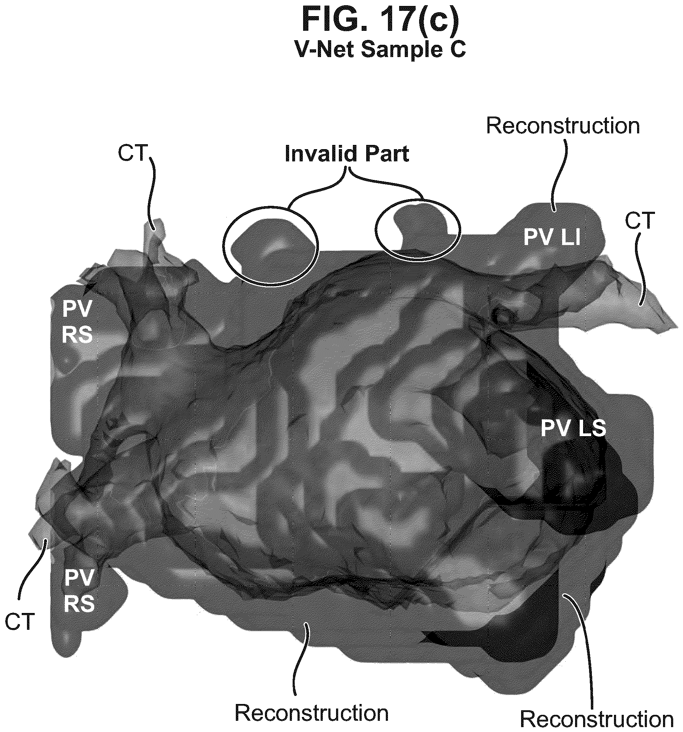

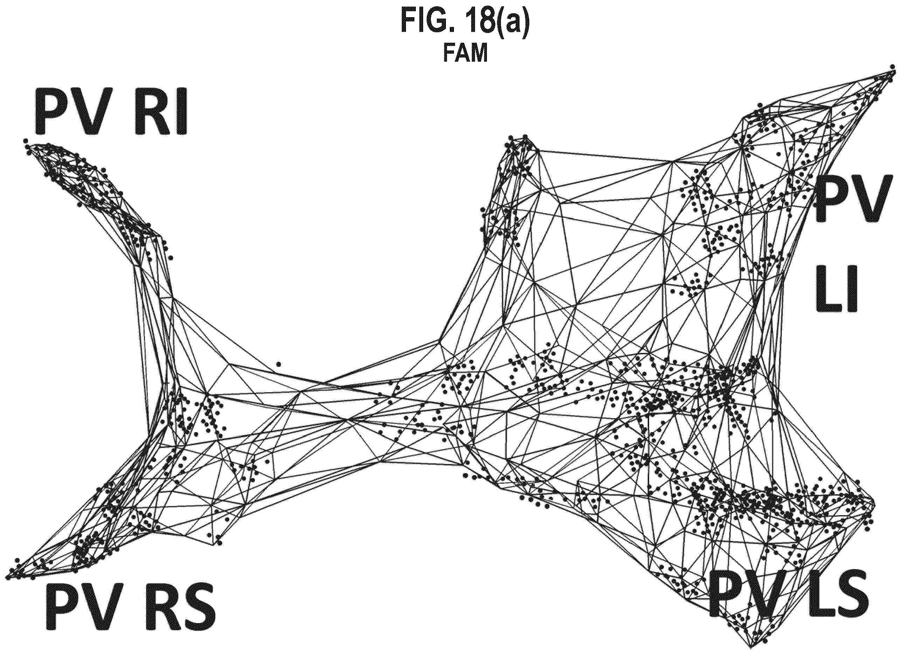

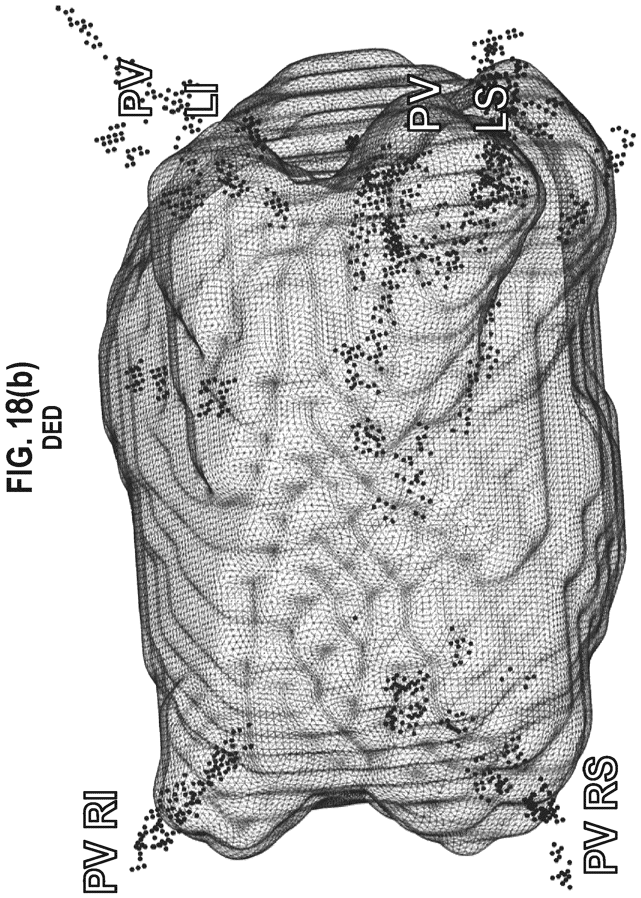

Qualitative results are shown in Figures 15 to 18. A comparison of the DED network reconstruction to the mean shape is shown in Figure 15 and Figure 16. Visualizations of V-Net reconstruction with anatomical issues are presented in Figure 17. Finally, we exemplify the advantage of using the proposed DED solution over the existing FAM system - for the 3 minutes point-set acquisition interval in Figure 18.

Figure 15 shows a comparison of reconstruction results focusing on the location and orientation of the PVs, for 2 sample cases. DED reconstruction is shown on top and the mean shape reconstruction is shown on the bottom. CT ground truth is shown in yellow, and the reconstruction results are in blue. Inspection reveals that the DED network improved the estimated PVs location and orientation. In particular, the mean shape reconstruction misses parts of the PVLS and PVRI.

Additional examples are shown in Figure 16. Here, four reconstruction methods (2 DED network solutions, V-Net, and the mean-shape reconstruction) are compared - showing the reconstruction as well as the surface-to-surface distances. Reconstruction results are shown in odd-numbered rows, with the ground-truth CT surfaces shown in even-numbered rows. On top of the surfaces, We present distance maps for a 15mm radius from PV ostia points and unbounded (as in Table 2). Distance maps measured from reconstruction to CT, are presented in odd-numbered rows, and a reverse visualization, from CT to reconstruction is shown in the even-numbered rows. We note that the errors are larger in the mean-shape reconstruction - with fewer dark blue regions (overall lighter color-scale). The right PVs are magnified to support the visualization of these regions in the three networks compared to the mean shape on the bottom.

In many of the cases experimented with (including many samples shown), the use of the V-Net solution resulted in a smooth reconstructed surface and gave low quantitative errors. However, a closer look at clinical examples reveals the presence of anatomical deformation in the reconstruction. Figure 17 displays a few such cases Three examples are shown in which anatomical structures are added or removed from the LA surface, as confirmed by the given CT anatomy.

Similar, minor to severe anomalies, were found in more than half of the 26 CT cases, thus making the value of V-Net for this task questionable.

Figure 18 compares the reconstruction results for FAM vs DED solution after acquiring the initial (3-minute interval) bearing path. In this case the FAM reconstruction is not anatomically viable while the DED reconstruction is anatomically correct.

## 5. Discussion

Our findings suggest that a neural-network based solution is capable of reconstructing the LA shape from a very sparse point cloud path. Although the well-known V-Net network exhibited superior performance in many of the quantitative metrics, the reconstructions contained many poorly represented parts of the anatomy, thus making it unsuitable as a clinical solution. Thus, the DED network regularized by a novel spatial weight smoothing and boundary enhancement map was the overall best performing architecture. The DED network solution learned smooth probable atrium parts and combined them to recover a realistic-looking left atrium.

Using real clinical cases enabled a clearer distinction of network performance:
When quantifying the performance error, we saw that the DED solution was consistent across various values for the SWR parameter; The reconstruction accuracy was not far behind the FAM performance and achieved statistically significant mean error improvement over the mean shape for the clinically relevant regions (CT evaluation), and contact points. The focus on clinically relevant regions and contact points is critical, since global metrics for the entire shape such as DICE, AVDist, and surface-to-surface distance, accumulate errors from many volume/surface points over the LA body. These constitute large regions with a small number of measurements that are subject to large variations due to registration and motion errors. Methods that fit well over the data could improve these global metrics while still generating ill-formed anatomy. In our analysis, we have full disclosure of gender information coming from one of the data source centers - indicating both male and female clinical cases analyzed. The gender distribution of clinical cases is missing in two other centers.

Note the inherent trade-off between fitting the input data and preserving its resemblance to the learned atrium anatomy. For the current task, we prefer networks that do not over-fit the data. This is likely to hold even for inputs that exhibit less resemblance to the data in the training set as seen in the clinical cases. DED without SWR introduced large uncertainty regions and non-anatomical structures to cope with paths less similar to those trained upon.

Qualitative inspection clearly revealed that the V-Net solution was not anatomically probable. It produced a smooth but twisted shape. This might be due to the lack of global understanding of the shape in this network since each convolution operation is local. This outcome is consistent with the findings in <<Z. Xiong, et al., Automatic 3d surface reconstruction of the left atrium from clinically mapped point clouds using convolutional neural networks, Front. physiology 13 (2022) 880260-880260, 35574484[pmid]>> in which the authors show that their V-Net-based solution performance depends on a large percent coverage of the atria surface (note that this requires a longer acquisition time, as in FAM).

The DED solution proposed combines two time intervals: time to acquire the path, and time for inference in which the LA is reconstructed. The first, path acquisition interval, is approximately 3 minutes. This enables an anatomically correct visualization with reasonable accuracy within a third of the acquisition time, as compared to current methods in the clinic (FAM). This early visualization simplifies the procedure, supports novel workflows, eases operator load, and may contribute to safety, especially for inexperienced physicians. The DED network inference is conducted in real-time.

6. Conclusion

In this study, we used a neural network-based approach to recover the shape of the left atrium from catheter paths. We used an anatomically based model to generate left atrium shapes and a novel algorithm to create paths inside them.

These paths were sufficient to teach the network to complete the left atrium shape from actual catheter paths in human cases.

The current work is part of an effort to build a system that infers the most probable anatomically correct reconstruction while considering currently avail able data. The data acquired in the clinic may contain additional information such as force measurements, electrocardiograms that hint at the anatomical region, etc. Models that can incorporate additional data and user inputs regarding the anatomy will further improve and simplify the mapping process.

The network was trained for the widespread anatomy of four PVs, and typical size variants. To deal with variations in anatomy, a model trained with the appropriate dataset should be used. The network approach, as proposed in this work, is a general approach that is likely to be applicable to other heart chambers and organs, different imaging techniques, and different types of partial inputs.

APPENDIX

A. Appendix

This section presents the details of the synthetic path creation algorithm.

A. 1. Marking points in the PV's interior for all atria

In this stage, for each input atrium, we found a point for every PV that is inside the ostium. We utilized the mean LA shape reference, having manually selected a point on each artificially closed PV.

The corresponding points on all the other LA samples were then generated by taking advantage of the fact that all atrial surfaces are similarly produced by the LA instance generator. These samples have Gaussian-like distribution for the orientations and the locations of the PVs. This step operated over the triangulated mesh representation to utilize the geometric properties of the surface. In the mesh representation, the chosen landmark points of the mean shape are vertices of the mesh.

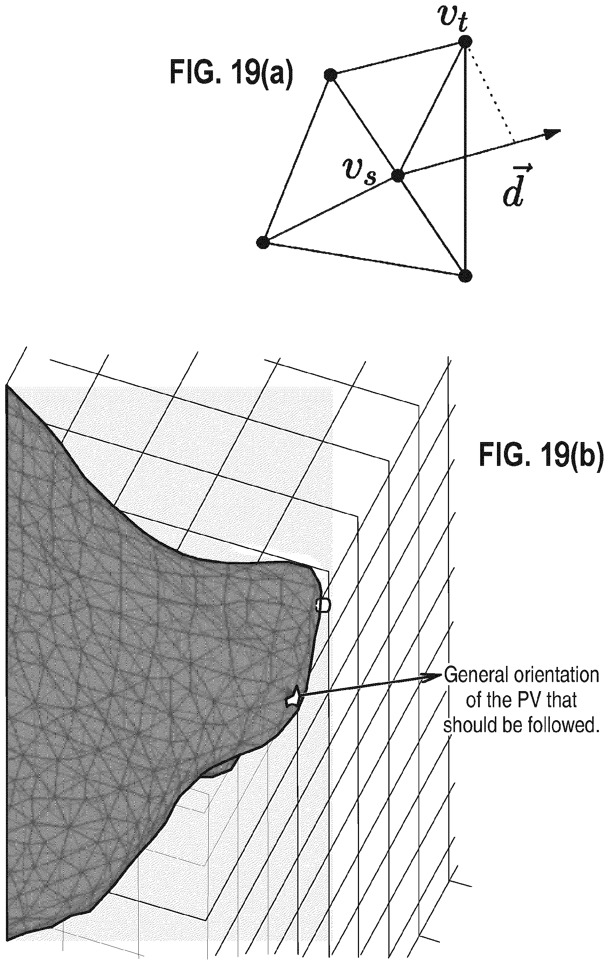

Given the input atrium, we found for each landmark vertex (of the mean shape), the closest vertex over the input LA mesh. For each vertex, the normal (to the surface) at the abovementioned vertex of the mean atria is used as a direction to 'slide' across the new atrium vertices from the initial vertex until we can no longer advance in the direction of the normal, as seen in Figure 19.

This vertex is likely to be on the same PV in the input atrium.

As shown in Algorithm 1 the input to the algorithm consists of the triangulated mesh (vertices and triplets of face membership) of the new atrium, a vertex p which is the original vertex in the mean atrium, a direction $\vec{d}$ which is the normal at p in the mean atrium and a threshold $\varepsilon$. In line 2 we assign the nearest vertex on the new atrium to the initial vertex from the mean atrium. Lines 4- 6 compute the direction vectors from the current vertex to its neighbors. In Lines 7 - 9 we assign the neighboring vertex whose direction vector projection over $\vec{d}$ is maximal. The loop terminates once the projected step length is less than $\varepsilon$.

The septum point is a bit different to locate as it is chosen clinically by the physician on the septal wall between the right and the left atria. We decided to simulate this by finding the nearest vertex to a chosen septum in the mean shape and then sampled a vertex (using a Gaussian distribution) around this vertex.

$$\text{FindPointInPV}\left(mesh, p, \vec{d}, \in\right)$$

$$current\ vertex \leftarrow FindNearestVertex\left(mesh, p\right)$$

$$\textbf{do}\ ($$

$$neighbouring\ vertices \leftarrow getNeighbours(mesh, current\ vertex)$$

$$\overrightarrow{dist\ vectors} \leftarrow neighbouring\ vertices - current\ vertex$$

$$\overrightarrow{dist\ vectors} \leftarrow \frac{dist\ vectors}{\|dist\ vectors\|}$$

$$direction\ difference \leftarrow \max \overrightarrow{dist\ vectors} \cdot \vec{d}$$

$$current\ vertex \leftarrow \arg max_{v\ \in\ neighbouring\ vertices} \overrightarrow{dist\ vectors} \cdot \vec{d})$$

$$\textbf{while}\ direction\ difference \geq \in;$$

**Algorithm 1**: Find PV Algorithm

A.2. Creating a Path between two PV's

A catheter maneuver between two points inside the left atrium is very different from a straight line due to the limited degrees of freedom of the catheter.

A realistic path between two PVs is relatively short and lies close to the center of the atrium (since a retraction to the center is usually performed while going from one area to the other). We express this problem by using a graph weighted such that the optimal path between two nodes will follow these two considerations. First, we use the discrete volume representation where voxels inside the atrium are represented by one. Next, we find the voxels that represent the two PVs. The graph is built such that each voxel in the volume is a node edge connected to its six neighbors. The boundary of the volume is extracted and a discrete signed distance transform (Euclidean distance) is computed over the volume.

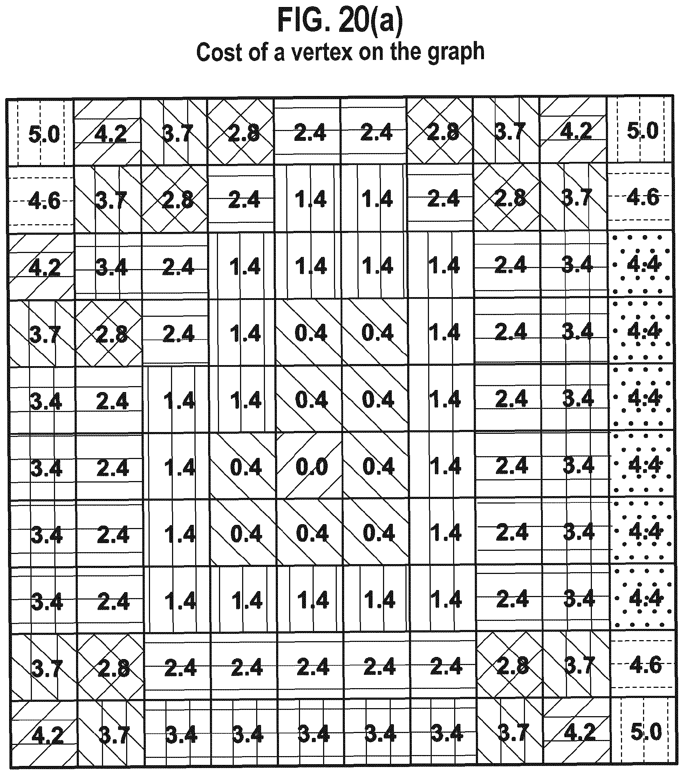

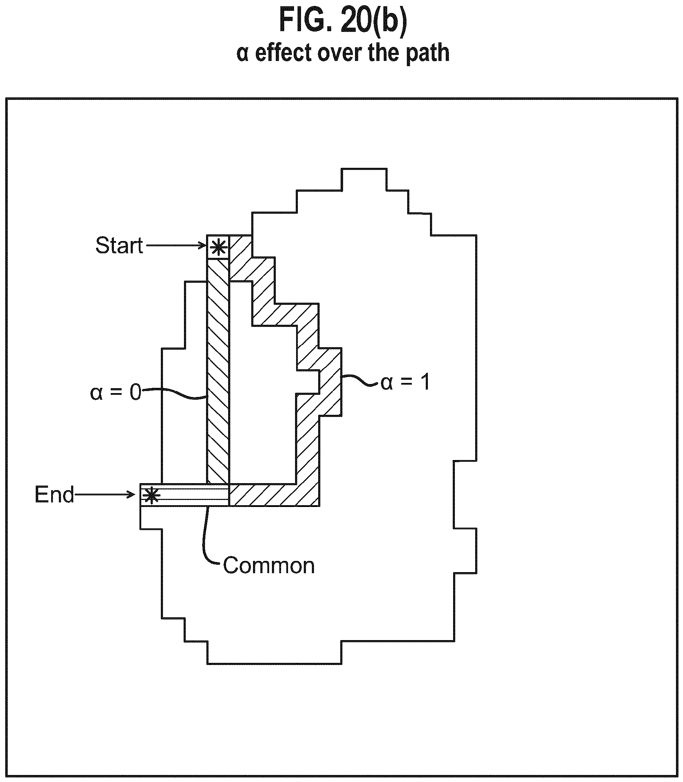

To compute this metric, a set of boundary voxels are selected having a distance of zero, and all other voxels are assigned a value that is the distance to the nearest boundary voxel. The value is negative for voxels outside the atrium, and positive inside. Thus, voxels close to the atrium center have high Euclidean distance values. To define a cost for minimization, we denote the maximal distance as mw and assign each voxel the weight $m_w$ - wat where wat is the voxel distance transform, as seen in Figure 20.

The edge between two neighboring voxels is assigned the mean of their weights. Edge cost increases as we move further away from the atrium's center. The shortest path between any two PVs is found using the Dijkstra algorithm (as described in T. H. Cormen, et al., Introduction to Algorithms, Third Edition, 3rd Edition, The MIT Press, 2009>>), with regard to our objectives favoring a short path (since each edge adds weight) that is close to the atrial center.

We also define an $\alpha$ parameter where each weight w becomes $w^{\alpha}$. When $\alpha$ reaches zero the path tends toward the shortest path (as each weight becomes one), whereas $\alpha$ progresses towards one and beyond the path tends towards the center, see Figure 20.

A.3. Path Part Integration

In order to create a full synthetic path we first we project the septum point from the template sample to the current sample. We then find point to point paths as described in Appendix A.2. The first path is from the septum to the left superior, the second continues to left inferior, the third to the right inferior and the last one to right superior; see Figure 8a. Different parts of the path can be equilibrated by using a different power squashing ($\alpha$) for each part, depending on how much we have to pull the catheter back to complete the path. The $\alpha$ parameter for each path was determined experimentally. The chosen values were [0.001, 4, 1,4] in the presented order. A sample path is shown in Figure 8b.Although the embodiments described herein mainly address cardiac applications, the methods and systems described herein can also be used in other medical applications, such as for producing three-dimensional models of other organs based on sparse data.

FINAL COMMENTS:

It will thus be appreciated that the embodiments and examples described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

**Claims**

1.  A computer-implemented method, comprising:

    receiving a first dataset of points acquired from a catheter as it follows a path across a heart, each point in the first dataset comprising position data acquired at a certain position along the path in the heart;
    providing the first dataset to an encoder-decoder network, the encoder-decoder network trained based on training data comprising (1) a dataset of points representing a shape of a known heart and (2) a dataset of points representing a known catheter path across the known heart;
    outputting, with the encoder-decoder network, a second dataset of points, the second dataset representing a predicted shape of the heart.

2.  A system comprising:

    a memory, which is configured to store a first dataset of points acquired from a catheter as it follows a path across a heart, each point in the dataset comprising position data acquired at a certain position along the path in the heart; and
    a processor which is configured to:

    receive, from the memory, the first dataset of points;
    provide the first dataset to an encoder-decoder network, the encoder-decoder network trained based on training data comprising (1) a dataset of points representing a shape of a known heart and (2) a dataset of points representing a known catheter path across the known heart;
    output, with the encoder-decoder network, a second dataset of points in the heart, the second dataset representing a predicted, shape of the heart.

3.  The method of claim 1, wherein the receiving the first dataset of points step comprises receiving the first dataset of points from a memory.

4.  The method of claim 1 or 3, or the system of claim 2, wherein the encoder-decoder network is a dense encoder-decoder network.

5.  The method of any one of claims 1, 3, and 4, the method further comprising reconstructing the predicted shape of the heart based on the predicted second dataset of points in the heart.

6.  The method of any one of claims 1 and 3 to 5, or the system of claim 2 or 4, wherein the path across the heart is a path across a left atrium, wherein the known catheter path across the known heart is a path across a known left atrium, and wherein the shape of the known heart is a shape of at least a portion of the known left atrium.

7.  The method of claim 6 or the system of claim 6, wherein the path and the known catheter path each follow a certain trajectory through the left atrium.

8.  The method of claim 7 or the system of claim 7, wherein each trajectory includes a path starting from the septum, and continuing to left inferior, right inferior, and right superior in order.

9.  The method of any one of claims 1 and 3 to 8, or the system of any one of claims 2, 4, and 6 to 8, wherein the training of the encoder-decoder network comprises minimizing a loss function, and wherein the loss function comprises a cross entropy loss term.

10. The method of claim 9 or the system of claim 9, wherein the loss function comprises a linear combination of the cross entropy loss term and a negative of a Sorenson-DICE coefficient.

11. The method of claim 10 or the system of claim 10, wherein the Sorenson-Dice coefficient is weighted with a weighting mask parameter, the weighting mask parameter configured to control the sensitivity of the model around the surface boundary of the heart.

12. The method of any of claims 9 to 11 or the system of any of claims 9 to 11, wherein the loss function further comprises a regularization term, the regularization term configured to control the smoothness of the shape of the heart.

13. The method of claim 12 or the system of claim 12, wherein the regularization function comprises derivatives of weights of a first layer of the encoder-decoder network, and preferably derivates of weights of the first layer and the output layer of the encoder-decoder network.

14. The method of any of claims 1 and 3 to 13, or the system of any of claims 2, 4, and 6 to 13, wherein the training of the encoder-decoder network comprises utilizing an Adam optimization algorithm.

15. The method of any of claims 1 and 3 to 14, or the system of any of claims 2, 4, and 6 to 13, wherein the encoder-decoder network comprises a first encoder and a first decoder, and wherein the encoder-decoder network comprises a plurality of layers, selected from one or more of an input layer, one or more hidden layers, and an output layer.

16. The method of claim 15, or the system of claim 15, wherein the encoder decoder network comprises one or more of the following:

> tied weights between the first encoder and the first decoder;
> a masked input;
> an RELU activation function in each layer in the encoder-decoder network except for the output layer;
> a sigmoid activation function in the output layer;
> a batch-normalization layer following each layer in the encoder-decoder network except for the output layer.

17. The method of any of claims 1 and 3 to 16, or the system of any of claims 2, 4, and 6 to 16, wherein at least one point in the dataset corresponds to a position in the blood-pool where the catheter is not touching the surface of the heart.

18. The method of claim 17 or the system of claim 17, wherein every point in the dataset corresponds to a position in the blood-pool where the catheter is not touching the surface of the heart.

19. The method of any of claims 1 and 3 to 18, or the system of any of claims 2, 4, and 6 to 18 wherein the training data dataset of points representing the known catheter path across the known heart comprises added noise and the trained encoder-decoder network is configured to remove noise from the first dataset of points.

20. The system of any of claims 2, 4, and 6 to 19, wherein the processor is further configured to reconstruct a shape of the heart based on the predicted second dataset of points in the heart.

FIG. 1

24

FIG. 2A

FIG.2B

FIG. 3

| Training phase 71 | Receive one or more example representations of geometrical shapes of left atria |
| | Train a neural network model using the one or more example representations |
| | Store parameters of trained neural network model in memory |

74

76

78

| Modeling phase 72 | Measure set of locations in left atrium |
| | Apply trained neural network model to measured set of locations |
| | Produce three-dimensional model of the left atrium |

84

86

88

*FIG. 4*

*FIG. 5*

FIG. 6(a)

EP 4 541 262 A1

FIG. 6(b)

FIG. 6(c)

## FIG. 7

Data Generation

Synthetic Data Generation

Left Arterial Shape          Path Generation

Volexised Path

45x45x45

Discretization

Catheter Traversal in Clinic

PV
RI

PV
LI

PV
RS

PV
LS

FIG. 7(contd.)

**LA Network Reconstruction**

Spatial Input Layer k=91250 — $W_1 (45^3 \times 350)$ — $W_1^T$ — Spatial Output Layer k=91250

Flatten

Hidden Layer k=350 — Hidden Layer k=350

k=350

$W_2$ $(350 \times 350)$ $W_3$ $(350 \times 350)$

DED

Unflatten

LA Reconstruction

EP 4 541 262 A1

FIG. 7(contd.)

EP 4 541 262 A1

FIG. 8(a)

PV LI

PV RI

PV LS

LAA

PV RS

Septum

⊘ Septum to PVLS    ⊖ PVLS to PVLI
Ⓘ PVLI to PVRI    ⊘ PVRI to PVRS

EP 4 541 262 A1

FIG. 8(b)

FIG. 8(c)

FIG. 9

FIG. 10(a)

FIG. 10(b)

## FIG. 11(a)

Occupied space

## FIG. 11(b)

High value region

## FIG. 12(a)
### Focal Catheter Point Cloud Path

**FIG.12(b)**
Focal Catheter Voxelized Path

FIG. 12(c)
Lasso Catheter Voxelized Path

## FIG. 12(d)

Point cloud of a focal catheter registered with the blue path of the mean shape.

PV RI

PV LI

PV

RS

PV LS

● Acquired path

○ Synthetic template path

EP 4 541 262 A1

# FIG. 13(a)

## Sample A

FIG. 13(b)
Sample B

# FIG. 14
## Comparison of SWR005 and mean shape surface to surface distance for several radii (mm).

# FIG. 14(contd.)
## Comparison of SWR005 and mean shape surface to surface distance for several radii (mm).

## FIG. 15(a)

## FIG. 15(b)

# FIG. 16

**Visualization of the reconstructions in the clinical CT cases for the SWR005, SWR75, V-Net , and the mean shape.**

## FIG. 16(contd.)
### Visualization of the reconstructions in the clinical CT cases for the SWR005, SWR75, V-Net , and the mean shape.

EP 4 541 262 A1

# FIG. 17(a)
## V-Net Sample A

# FIG. 17(b)
## V-Net Sample B

# FIG. 17(c)
## V-Net Sample C

FIG. 18(a)
FAM

FIG. 18(b)

FIG. 19(a)

$v_t$

$v_s$

$\vec{d}$

FIG. 19(b)

General orientation
of the PV that
should be followed.

# FIG. 20(a)
## Cost of a vertex on the graph

| 5.0 | 4.2 | 3.7 | 2.8 | 2.4 | 2.4 | 2.8 | 3.7 | 4.2 | 5.0 |
|------|------|------|------|------|------|------|------|------|------|
| 4.6 | 3.7 | 2.8 | 2.4 | 1.4 | 1.4 | 2.4 | 2.8 | 3.7 | 4.6 |
| 4.2 | 3.4 | 2.4 | 1.4 | 1.4 | 1.4 | 1.4 | 2.4 | 3.4 | 4.4 |
| 3.7 | 2.8 | 2.4 | 1.4 | 0.4 | 0.4 | 1.4 | 2.4 | 3.4 | 4.4 |
| 3.4 | 2.4 | 1.4 | 1.4 | 0.4 | 0.4 | 1.4 | 2.4 | 3.4 | 4.4 |
| 3.4 | 2.4 | 1.4 | 0.4 | 0.0 | 0.4 | 1.4 | 2.4 | 3.4 | 4.4 |
| 3.4 | 2.4 | 1.4 | 0.4 | 0.4 | 0.4 | 1.4 | 2.4 | 3.4 | 4.4 |
| 3.4 | 2.4 | 1.4 | 1.4 | 1.4 | 1.4 | 1.4 | 2.4 | 3.4 | 4.4 |
| 3.7 | 2.8 | 2.4 | 2.4 | 2.4 | 2.4 | 2.4 | 2.8 | 3.7 | 4.6 |
| 4.2 | 3.7 | 3.4 | 3.4 | 3.4 | 3.4 | 3.4 | 3.7 | 4.2 | 5.0 |

# FIG. 20(b)
## α effect over the path

Europäisches
Patentamt

European
Patent Office

Office européen
des brevets

# EUROPEAN SEARCH REPORT

Application Number

EP 23 20 3849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIONG ZHAOHAN ET AL: "Automatic 3D Surface Reconstruction of the Left Atrium From Clinically Mapped Point Clouds Using Convolutional Neural Networks", FRONTIERS IN PHYSIOLOGY, vol. 13, 1 April 2022 (2022-04-01), XP093135113, CH ISSN: 1664-042X, DOI: 10.3389/fphys.2022.880260 * pages 3-5; figures 3, 4 * | 1-20 | INV. A61B5/00 G16H30/40 |
| Y | US 2020/029845 A1 (BARAM ALON [IL] ET AL) 30 January 2020 (2020-01-30) * paragraphs [0033] - [0049]; figures 1-4 * | 1-20 | |
| Y | BEETZ MARCEL ET AL: "Point2Mesh-Net: Combining Point Cloud and Mesh-Based Deep Learning for Cardiac Shape Reconstruction", 28 January 2023 (2023-01-28), 20230128, PAGE(S) 280 - 290, XP047646963, [retrieved on 2023-01-28] * pages 281-284; figures 1-3 * | 1-20 | |
| A | BARAM ALON ET AL: "Left Atria Reconstruction from a Series of Sparse Catheter Paths Using Neural Networks", 12 September 2018 (2018-09-12), MACHINE LEARNING FOR MEDICAL IMAGE RECONSTRUCTION; [LECTURE NOTES IN COMPUTER SCIENCE; LECT.NOTES COMPUTER], SPRINGER INTERNATIONAL PUBLISHING, CHAM, PAGE(S) 138 - 146, XP047669140, ISSN: 0302-9743 ISBN: 978-3-030-00128-5 [retrieved on 2018-09-12] * pages 140-141 * | 1,2,9-14 | |

**TECHNICAL FIELDS SEARCHED (IPC)**

A61B
G16H

The present search report has been drawn up for all claims

| Place of search | Date of completion of the search | Examiner |
|---|---|---|
| Berlin | 28 February 2024 | Dydenko, Igor |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 20 3849

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2024

| Patent document cited in search report | Publication date | Patent family member(s) | Publication date |
|---|---|---|---|
| US 2020029845 A1 | 30-01-2020 | AU 2019205023 A1 | 13-02-2020 |
| | | CA 3050653 A1 | 30-01-2020 |
| | | CN 110782520 A | 11-02-2020 |
| | | EP 3605391 A1 | 05-02-2020 |
| | | IL 268081 A | 30-01-2020 |
| | | JP 7366623 B2 | 23-10-2023 |
| | | JP 2020021488 A | 06-02-2020 |
| | | US 2020029845 A1 | 30-01-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140152653 A
- US 20170046616
- US 9576107 B
- US 8456182 B

**Non-patent literature cited in the description**

- **VINCENT et al.** Extracting and Composing Robust Features with Denoising Autoencoders. *Proceedings of the 25th ACM International Conference on Machine Learning*, 2008, 1096-1103
- **L. SCIARRA et al.** Utility of newly available carto 3 mapping system to guide catheter ablation of atrial fibrillation. *EUROPEAN HEART JOURNAL*, vol. 31
- **E. AHMED et al.** Deep learning advances on different 3d data representations: A survey. *CoRR abs/1808.01462*
- **A. BROCK et al.** Generative and discriminative voxel modeling with convolutional neural networks. *arXiv:1608.04236*
- **C. CHEN et al.** Deep learning for cardiac image segmentation: A review. *Front. Cardiovasc. Med.*, 7
- **M. AVENDI et al.** A combined deep-learning and deformable-model approach to fully automatic segmentation of the left ventricle in cardiac mri. *Med. Image Anal*, 2016, vol. 30, 108-119
- **H. LIAO et al.** More knowledge is better: Cross-modality volume completion and 3D+2D segmentation for intracardiac echocardiography contouring. *Lecture Notes in Computer Science (including subseries Lecture Notes in Artificial Intelligence and Lecture Notes in Bioinformatics)*, 2018
- **«F. MILLETARI et al.** V-Net: Fully convolutional neural networks for volumetric medical image segmentation. *Proceedings - 2016 4th International Conference on 3D Vision, 3DV 2016*, 2016
- **Z. XIONG et al.** Automatic 3d surface reconstruction of the left atrium from clinically mapped point clouds using convolutional neural networks. *Front. physiology*, 2022, vol. 13, 880260-880260
- **F. MILLETARI et al.** V-Net: Fully convolutional neural networks for volumetric medical image segmentation. *Proceedings - 2016 4th International Conference on 3D Vision, 3DV 2016*, 2016
- **T. H. CORMEN et al.** Introduction to Algorithms. The MIT Press, 2009
- Extracting and composing robust features with denoising autoencoders. **P. VINCENT et al.** Proceedings of the 25th international conference on Machine learning. ACM, 2008, 1096-1103
- **D. P. KINGMA** ; **J. BA, ADAM**. *A method for stochastic optimization*, 2014
- **S. IOFFE** ; **C. SZEGEDY**. Batch normalization: Accelerating deep network training by reducing internal covariate shift. *Proceedings of the 32nd International Conference on International Conference on Machine Learning*, 2015, vol. 37, 448-456
- **M. KASS** ; **A. WITKIN** ; **D. TERZOPOULOS**. Snakes: Active contour models. *Int. journal computer vision*, 1988, vol. 1 (4), 321-331
- Left atria reconstruction from a series of sparse catheter paths using neural networks. **A. BARAM et al.** Machine Learning for Medical Image Reconstruction - First International Workshop, MLMIR 2018, Held in Conjunction with MICCAI 2018, Proceedings, Lecture Notes in Computer Science (including subseries Lecture Notes in Artificial Intelligence and Lecture Notes in Bioinformatics). Springer Verlag, 2018, 138-146
- **O. RONNEBERGER et al.** U-net: Convolutional networks for biomedical image segmentation. *Lecture Notes in Computer Science (including subseries Lecture Notes in Artificial Intelligence and Lecture Notes in Bioinformatics)*, 2015
- **M. U. DALMI,S et al.** Using deep learning to segment breast and fibroglandular tissue in MRI volumes. *Med. Phys.arXiv:0608246v3*
- **Z. QUO et al.** Deep LOGISMOS: Deep learning graph-based 3D segmentation of pancreatic tumors on CT scans. *Proceedings - International Symposium on Biomedical Imaging*, 2018
- **A. MYRONENKO** ; **X. SONG**. Point set registration: Coherent point drift. *IEEE Trans. on Pattern Anal. Mach. Intell.*, 2010, vol. 32 (12), 2262-2275

- **K. H. CHA et al.** Urinary bladder segmentation in CT urography using deep-learning convolutional neural network and level sets. *Med. Phys.*, 2016, vol. 43 (4), 1882-1896

- **W. E. LORENSEN** ; **H. E. CLINE**. Marching cubes: A high resolution 3d surface construction algorithm. *SIGGRAPH Comput. Graph.*, 1987, vol. 21 (4), 163-169